# EUROPEAN PATENT APPLICATION

(11) **EP 4 603 090 A1**
(43) Date of publication of application: **20.08.2025**
(21) Application number: 25186872.5
(22) Date of filing: 08.11.2019
(51) Int. Cl.: A61K 31/341, A61K 38/22, C07K 14/58, A61P 9/12, A61P 13/12

(54) **COMBINATION TREATMENT FOR RESISTANT HYPERTENSION**

(30) Priority: 09.11.2018 US 201862758009 P
(62) Divisional of application: 19881216.6
(71) Applicant: Mayo Foundation for Medical Education and Research, Rochester, MN 55905 (US)
(72) Inventor: Burnett, John, C., Jr., Rochester, Minnesota 55902, (US); Dzhoyashvili, Nina, Rochester, Minnesota 55904 (US)
(74) Representative: Uexküll & Stolberg

(57) **Abstract**

Materials and methods for treating hypertension (including resistant hypertension) with a combination of an M-atrial natriuretic peptide (MANP) and a diuretic agent (e.g., furosemide) are described herein.

## Description

### CROSS-REFERENCE TO RELATED APPLICATIONS

This application claims benefit of priority from U.S. Provisional Application Serial No. 62/758,009, filed on November 9, 2018. The disclosure of the prior application is considered part of (and is incorporated by reference in) the disclosure of this application.

### STATEMENT OF FEDERALLY SPONSORED RESEARCH

This invention was made with government support under HL136340 awarded by the National Institutes of Health. The government has certain rights in the invention.

### TECHNICAL FIELD

This document relates to materials and methods for treating hypertension, and more particularly to methods for using a combination of an M-atrial natriuretic peptide (MANP) and a diuretic (e.g., furosemide) to treat mammals with resistant hypertension.

### BACKGROUND

Hypertension (HT), also known as high blood pressure, is a long term medical condition in which blood pressure in the arteries is persistently elevated. Hypertension is becoming increasingly resistant to treatment with anti-hypertensive drugs. Patients with resistant hypertension (RH) have blood pressure that remains elevated despite concurrent treatment with three antihypertensive agents of different classes, including (1) a diuretic and, typically, (2) an angiotensin-converting enzyme (ACE) inhibitor or angiotensin II receptor blocker (ARB) and (3) a calcium channel blocker (CCB). Patients whose blood pressure is controlled with four or more medications also are considered to have RH. There are currently no drugs or devices approved in the United States for treating RH.

### SUMMARY

This document is based, at least in part, on the discovery that the combination of an MANP and a diuretic has potent blood pressure (BP) lowering action while suppressing Fs-induced aldosterone stimulation. This discovery indicated that RH can be effectively treated with a combination of an MANP and a diuretic such as furosemide (Fs).

In a first aspect, this document features a method for treating a mammal, where the method can include administering to the mammal an MANP and a diuretic agent. The mammal can be identified as having hypertension (e.g., RH). The MANP can have the amino acid sequence set forth in SEQ ID NO:3, or the amino acid sequence set forth in any one of SEQ ID NOS:5 to 14. The diuretic agent can be furosemide. The mammal can be a human. The MANP can be administered intravenously (e.g., at a dose of about 10 pmol/kg/minute to about 100 nmol/kg/minute). The MANP can be administered subcutaneously (e.g., at a dose of about 0.1 ng/kg to about 10 mg/kg). The MANP can be administered intravenously and subsequently administered subcutaneously. For example, the MANP can be administered intravenously at a dose of about 10 pmol/kg/minute to about 100 nmol/kg/minute, and subsequently administered subcutaneously at a dose of about 0.1 ng/kg to about 100 mg/kg. The diuretic agent can be administered orally, intravenously, or subcutaneously. The MANP and the diuretic agent can be administered simultaneously in the same composition. The MANP and the diuretic agent can be administered subcutaneously. The method can consist of administering the MANP and the diuretic agent to the mammal. The MANP and the diuretic agent can be administered in a composition in which the sole active ingredients are the MANP and the diuretic agent.

In another aspect, this document features a method for potentiating one or more beneficial effects of a diuretic agent and reducing one or more detrimental effects of the diuretic agent in a mammal, where the method includes administering to the mammal the diuretic agent and an MANP. The mammal can be identified as having hypertension (e.g., RH). The MANP can have the amino acid sequence set forth in SEQ ID NO:3 or the amino acid sequence set forth in any one of SEQ ID NOS:5 to 14. The diuretic agent can be furosemide. The mammal can be a human. The MANP the diuretic agent can be administered intravenously (e.g., where the MANP is administered at a dose of about 10 pmol/kg/minute to about 100 nmol/kg/minute). The MANP and the diuretic agent can be administered subcutaneously (e.g., where the MANP is administered at a dose of about 1 µg/kg to about 10 µg/kg). The MANP and the diuretic agent can be administered intravenously and subsequently administered subcutaneously. The MANP can be administered intravenously at a dose of about 10 pmol/kg/minute to about 100 nmol/kg/minute, and subsequently administered subcutaneously at a dose of about 1 µg/kg to about 10 µg/kg. The one or more beneficial effects can include a reduction in blood pressure in the mammal, and the one or more detrimental effects can include aldosterone activation. The method can consists of administering a composition in which the sole active ingredients are the diuretic and the MANP. The MANP and the diuretic agent can be administered in a composition in which the sole active ingredients are the MANP and the diuretic agent.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF DRAWINGS

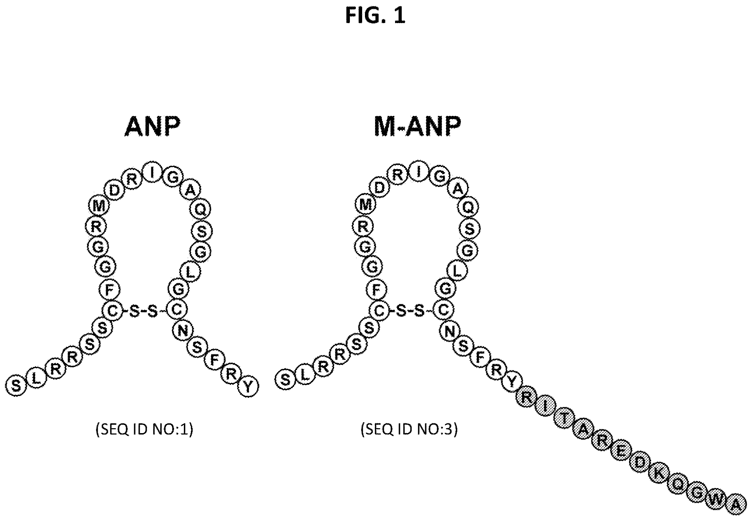

**FIG. 1** is a diagram showing the sequence and general structure of atrial natriuretic peptide (ANP; SEQ ID NO:1), as well as the sequence an general structure of an MANP (SEQ ID NO:3).

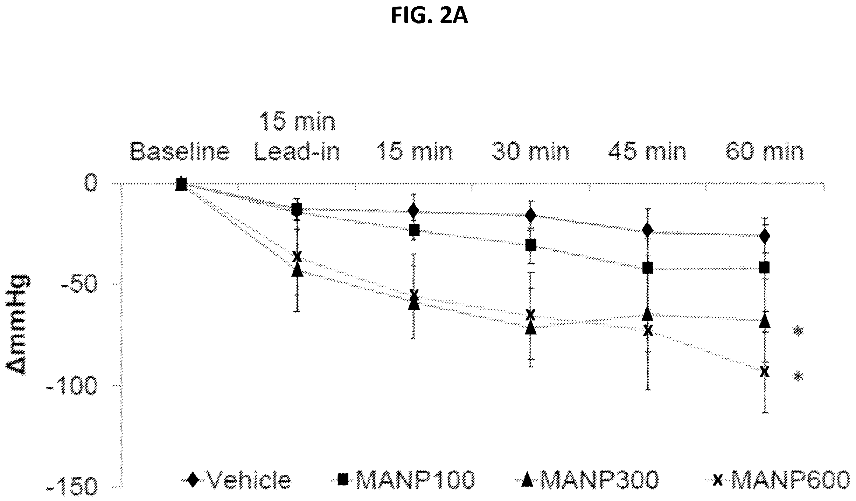

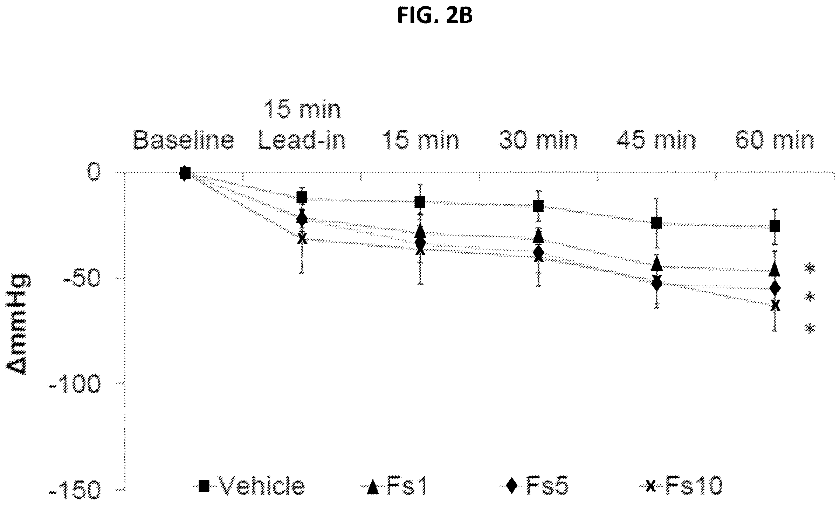

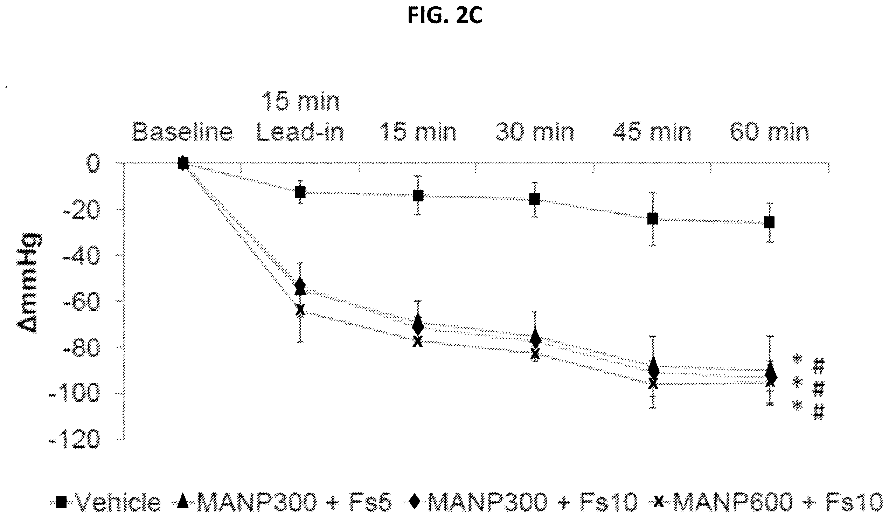

**FIGS. 2A-2C** are graphs plotting mean arterial pressure (MAP) in rats treated with vehicle, 100 pmol/kg/min MANP, 300 pmol/kg/min MANP, or 600 pmol/kg/min MANP **(****FIG. 2A****);** vehicle, 1 mg/kg Fs, 5 mg/kg Fs, or 10 mg/kg Fs **(****FIG. 2B****),** and vehicle, 300 pmol/kg/min MANP + 5 mg/kg Fs, 600 pmol/kg/min MANP + 5 mg/kg Fs, or 600 pmol/kg/min MANP + 10 mg/kg Fs **(****FIG. 2C****),** over a 60 minute time period. The MANP had the amino acid sequence set forth in SEQ ID NO:3. Data are presented as mean ± SD. *p<0.05 vs. Veh, ^{#}p<0.05 vs Fs10.

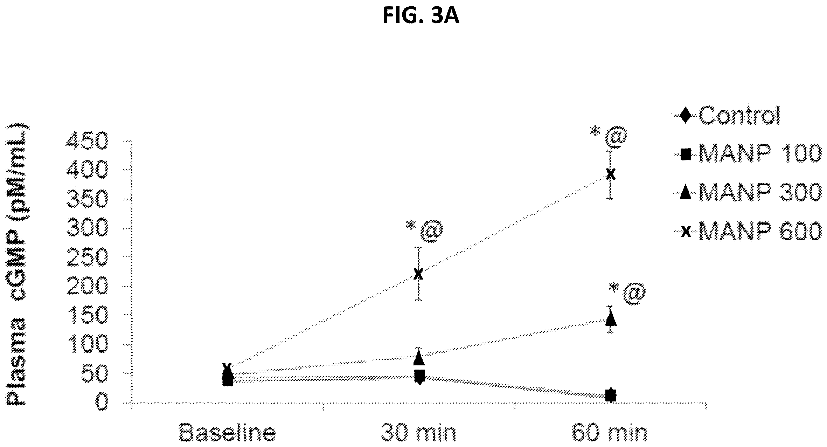

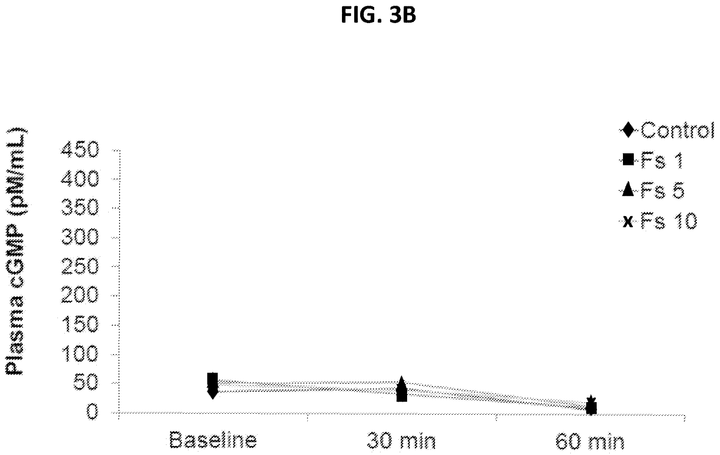

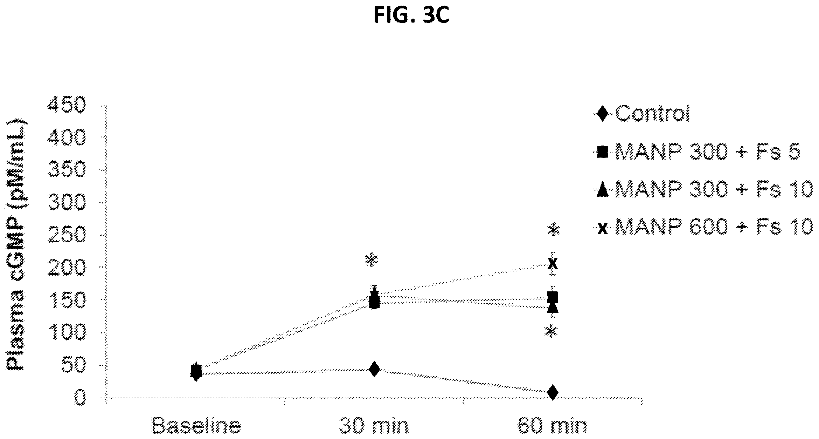

**FIGS. 3A-3C** are graphs plotting plasma cGMP levels in rats treated with vehicle, 100 pmol/kg/min MANP, 300 pmol/kg/min MANP, or 600 pmol/kg/min MANP **(****FIG. 3A****);** vehicle, 1 mg/kg Fs, 5 mg/kg Fs, or 10 mg/kg Fs **(****FIG. 3B****),** and vehicle, 300 pmol/kg/min MANP + 5 mg/kg Fs, 600 pmol/kg/min MANP + 5 mg/kg Fs, or 600 pmol/kg/min MANP + 10 mg/kg Fs **(****FIG. 3C****),** over a 60 minute time period. The MANP had the amino acid sequence set forth in SEQ ID NO:3. Data are presented as mean ± SD. *p<0.05 vs. vehicle @ p<0.05 vs. MANP100.

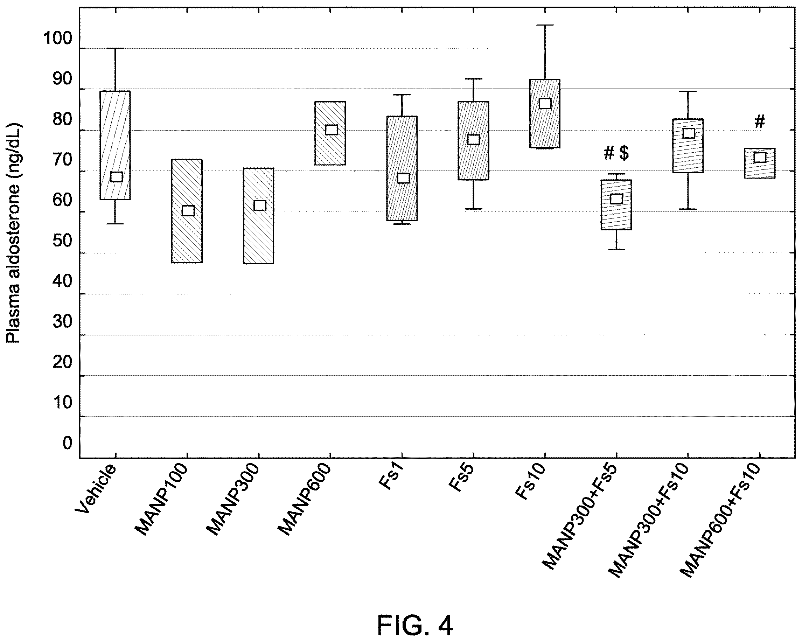

**FIG. 4** is a graph plotting plasma aldosterone levels in rats treated with vehicle, 100 pmol/kg/min MANP, 300 pmol/kg/min MANP, 600 pmol/kg/min MANP, 1 mg/kg Fs, 5 mg/kg Fs, 10 mg/kg Fs, 300 pmol/kg/min MANP + 5 mg/kg Fs, 600 pmol/kg/min MANP + 5 mg/kg Fs, or 600 pmol/kg/min MANP + 10 mg/kg Fs, for 60 minutes. The MANP had the amino acid sequence set forth in SEQ ID NO:3. Data are presented as mean ± SD. $p<0.05 vs. Fs5, # p<0.05 vs Fs10.

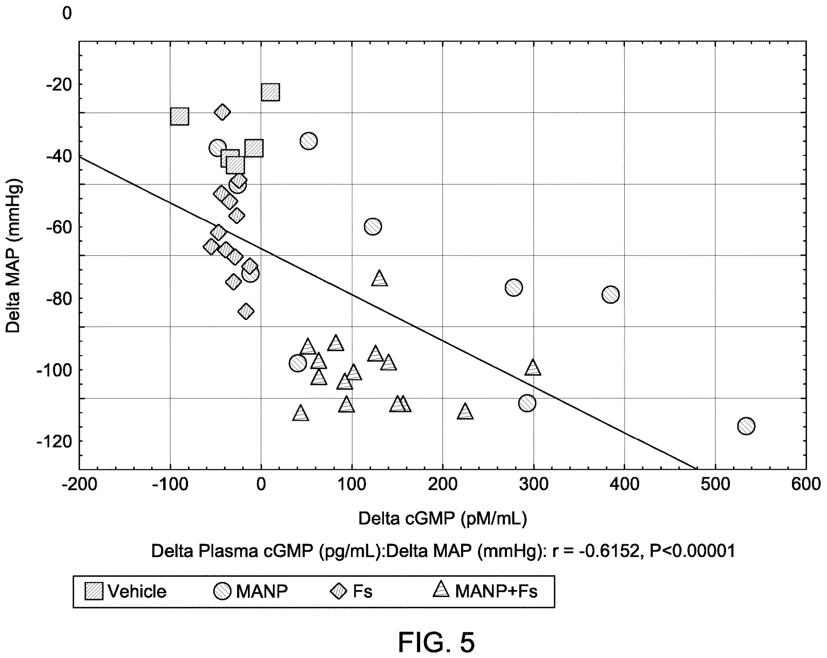

**FIG. 5** is a graph plotting the change in MAP vs. the change in cGMP levels after treatment of rats with vehicle, 100, 300, or 600 pmol/kg/min MANP, 1, 5, or 10 mg/kg Fs, or 300 pmol/kg/min MANP + 5 mg/kg Fs, 300 pmol/kg/min MANP + 10 mg/kg Fs, or 600 pmol/kg/min MANP + 10 mg/kg Fs for 60 minutes. The MANP had the amino acid sequence set forth in SEQ ID NO:3.

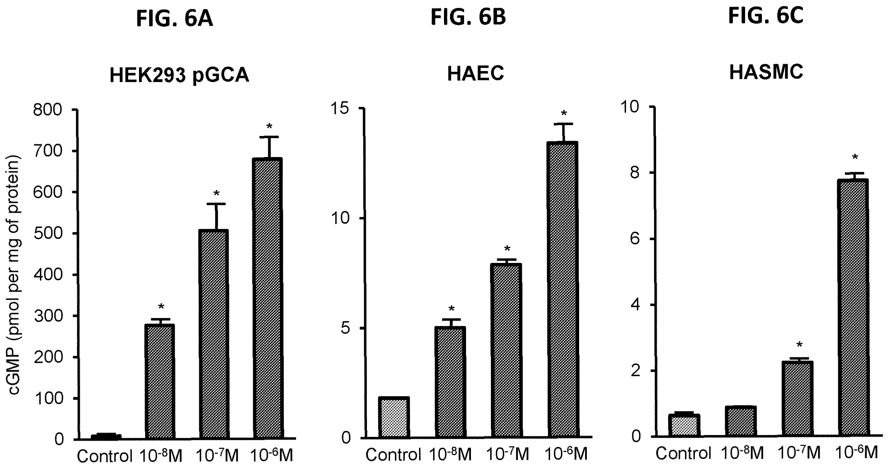

**FIGS. 6A-6C** are graphs plotting levels of cGMP generated in human embryonic kidney 293 (HEK293) cells overexpressing particulate guanylyl cyclase A (pGC-A) receptors **(****FIG. 6A****),** primary human aortic endothelial cells (HAECs) **(****FIG. 6B****),** and primary human aortic smooth muscle cells (HASMCs) **(****FIG. 6C****)** stimulated by MANP (SEQ ID NO:3). Values shown are mean = SEM. * p< 0.05 vs. control.

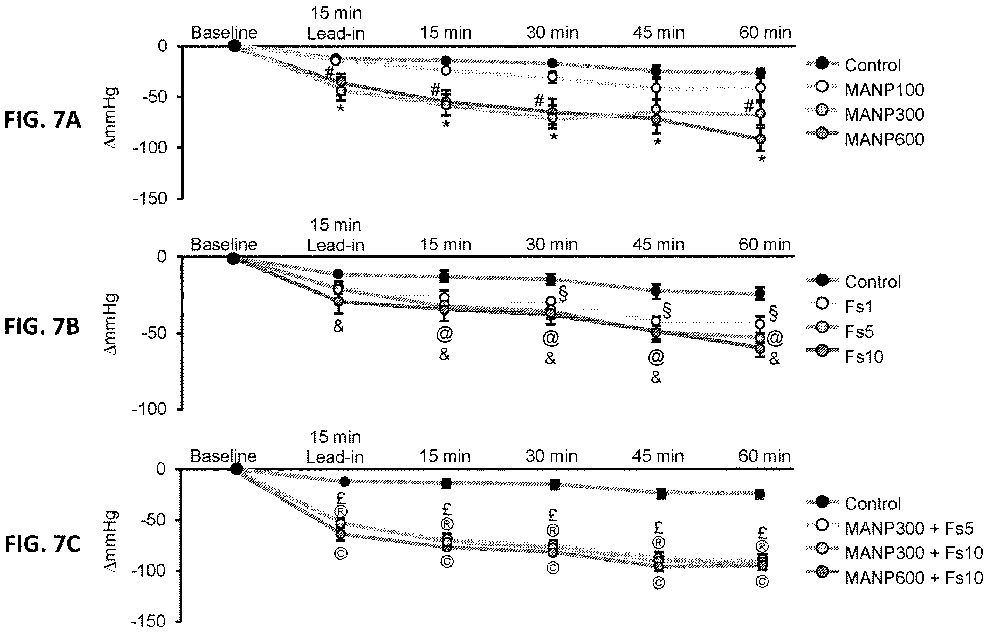

**FIGS. 7A-7C** are graphs plotting BP in rats at baseline (BL), 15 minute Lead-In, and 15, 30, 45 and 60 minutes after MANP or vehicle (control) infusion **(****FIG. 7A****),** after Fs or vehicle (control) bolus injection **(****FIG. 7B****),** and after a combination of MANP infusion and Fs bolus injection or vehicle infusion and vehicle bolus injection (control) **(****FIG. 7C****).** Values shown are mean ± SEM. # p< 0.05 MANP300 vs. vehicle; * p<0.05 MANP600 vs. vehicle; § p<0.05 Fs1 vs. vehicle; @ p<0.05 Fs5 vs. vehicle; & p<0.05 Fs10 vs. vehicle; £ p<0.05 MANP300+Fs5 vs. vehicle; ^{®} p<0.05 MANP300+Fs10 vs. vehicle; ^{©} p<0.05 MANP600+Fs10 vs. vehicle. The MANP had the amino acid sequence set forth in SEQ ID NO:3.

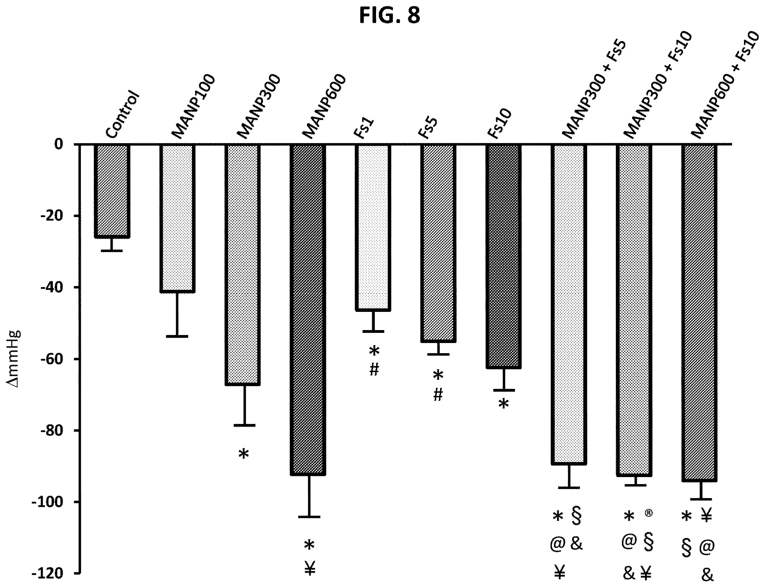

**FIG. 8** is a graph plotting BP in rats after 60 minutes of vehicle infusion (control), MANP infusion, Fs bolus injection, or a combination of MANP infusion and Fs bolus injection. Values plotted are mean ± SEM. * p< 0.05 vs. vehicle; ¥ p< 0.05 vs. MANP100; ^{®} p<0.05 MANP300 vs MANP300+Fs10; # p<0.05 vs MANP600; § p<0.05 vs. Fs1; @ p<0.05 vs Fs5; & p<0.05 vs Fs10. The MANP had the amino acid sequence set forth in SEQ ID NO:3.

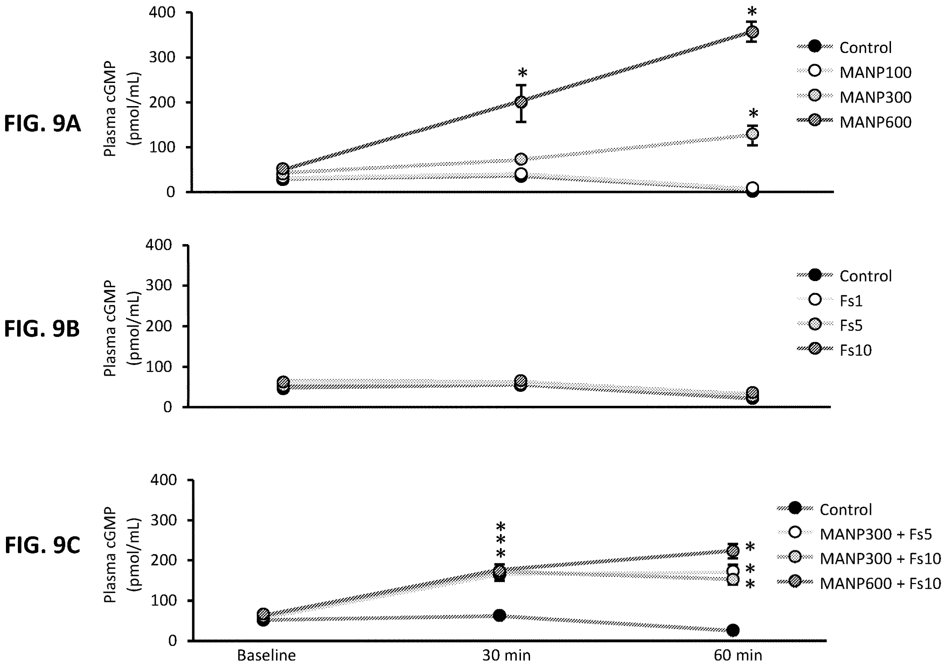

**FIGS. 9A-9C** are graphs plotting plasma cGMP in rats at BL, 30 and 60 minutes after MANP or vehicle (control) infusion **(****FIG. 9A****),** after Fs or vehicle (control) bolus injection **(****FIG. 9B****),** and after a combination of MANP infusion and Fs bolus injection or vehicle infusion and vehicle bolus injection (control) **(****FIG. 9C****).** Values plotted are mean ± SEM. * p< 0.05 vs. vehicle. The MANP had the amino acid sequence set forth in SEQ ID NO:3.

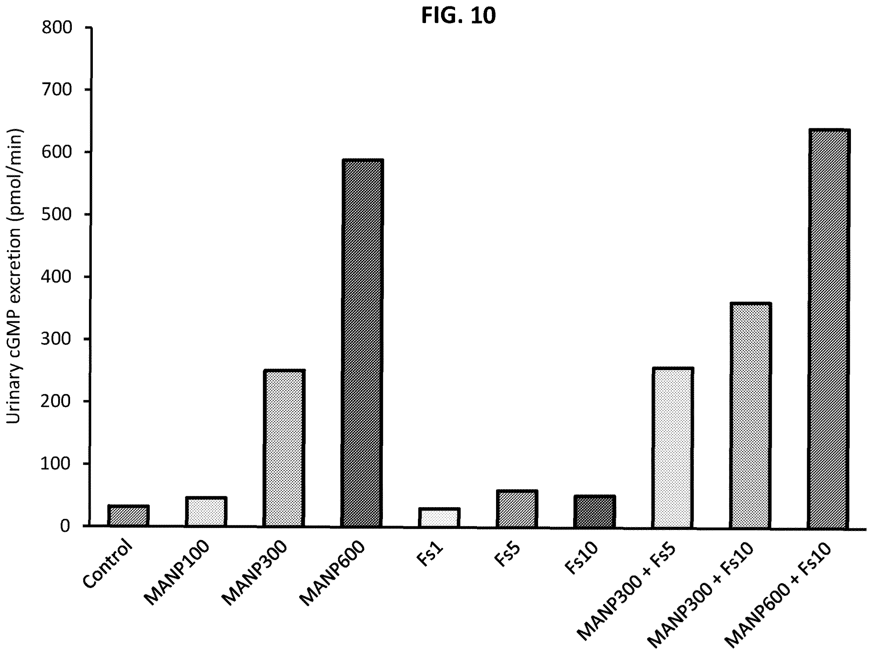

**FIG. 10** is a graph plotting urinary cGMP excretion in rats 60 minutes after administration of MANP (SEQ ID NO:3), Fs, combination MANP+Fs, or vehicle. Values plotted are mean ± SEM. * p< 0.05 vs. vehicle (control).

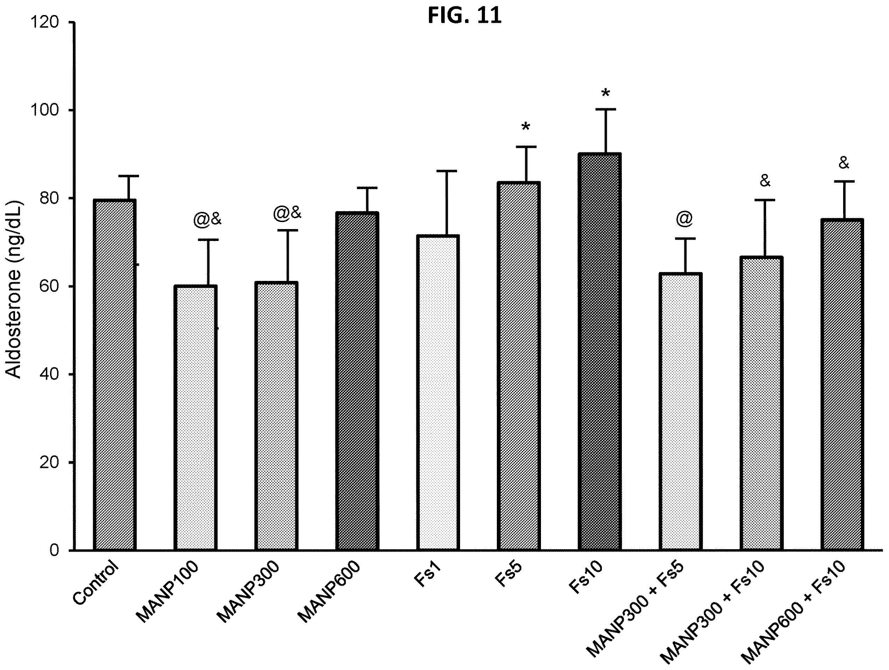

**FIG. 11** is a graph plotting aldosterone levels in rats 60 minutes after administration of MANP (SEQ ID NO:3), Fs, a combination of MANP+Fs, or vehicle. Values shown are mean ± SEM. * p< 0.05 vs. vehicle (control); @ p<0.05 vs Fs5; & p<0.05 vs Fs10.

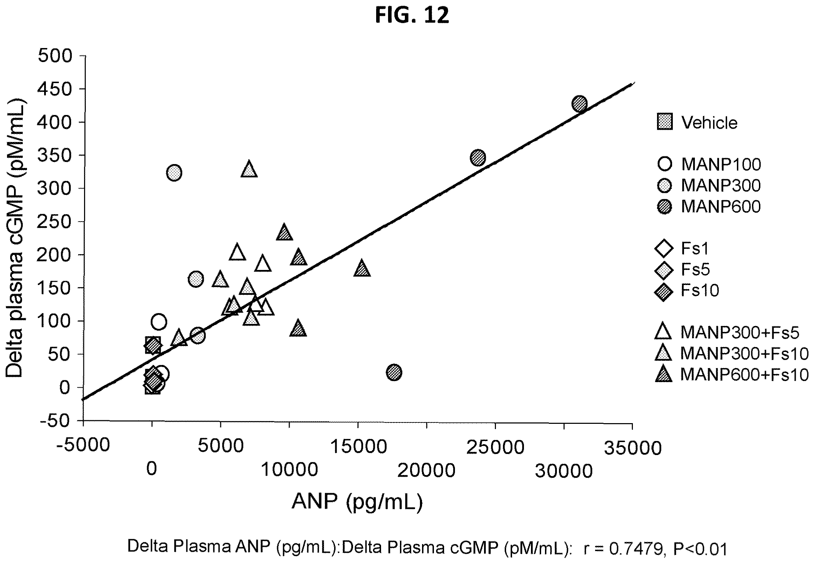

**FIG. 12** is a graph showing the positive correlation between plasma ANP and plasma cGMP in rats after administration of MANP (SEQ ID NO:3), Fs, a combination of MANP+Fs, or vehicle. Pearson's correlation coefficient (r) = 0.7479, p< 0.01.

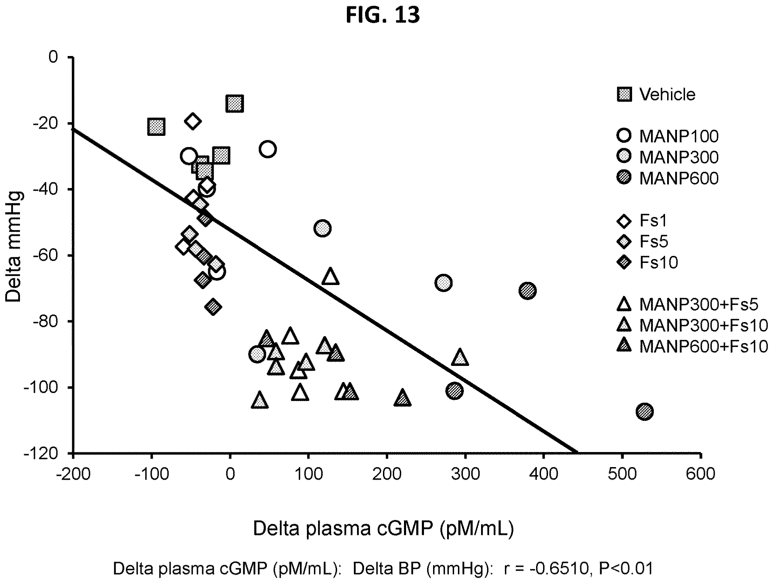

**FIG. 13** is a graph plotting the negative correlation between plasma cGMP and mean arterial blood pressure in rats after administration of MANP (SEQ ID NO:3), Fs, a combination of MANP+Fs, or vehicle. Pearson's correlation coefficient (r) = -0.6510, p< 0.01.

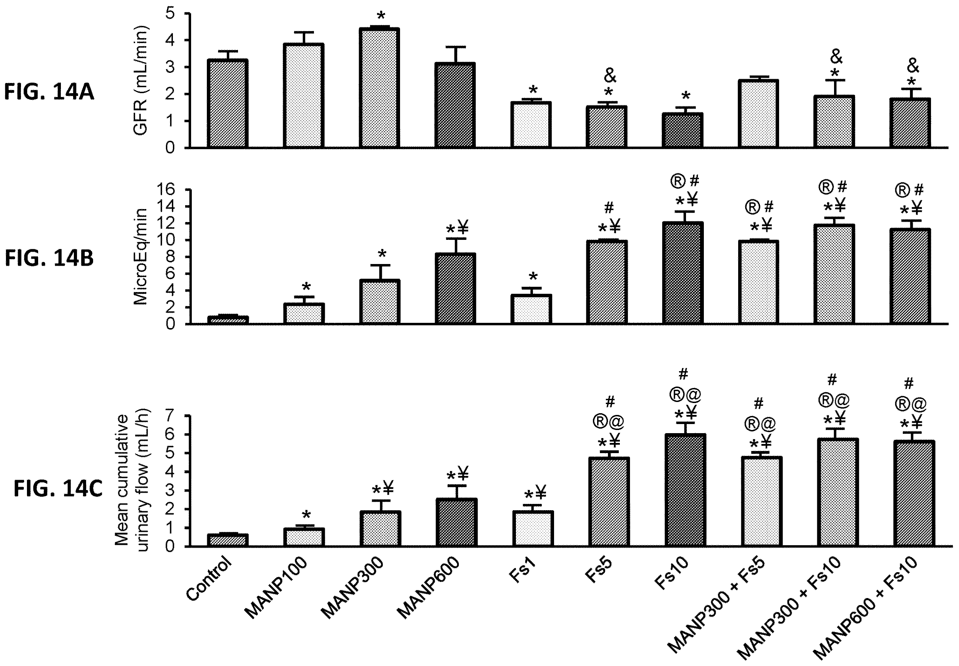

**FIGS. 14A-14C** are graphs plotting GFR **(****FIG. 14A****),** urinary sodium excretion **(****FIG. 14B****) and** urinary flow **(****FIG. 14C****)** 60 minutes after administration of MANP (SEQ ID NO:3), Fs, a combination of MANP+Fs, or vehicle. Values shown are mean ± SEM. * p< 0.05 vs. vehicle (control); ¥ p< 0.05 vs. MANP100; ^{®} p<0.05 vs. MANP300; @ p<0.05 vs MANP600; # p<0.05 vs Fs1; & p<0.05 vs MANP300+Fs5.

### DETAILED DESCRIPTION

This document provides materials and methods for treating hypertension, including RH, with a combination of an MANP and a diuretic (e.g., Fs). For example, this document provides methods that include administering an MANP and a diuretic to a mammal (e.g., a human, non-human primate, dog, cat, rat, mouse, cow, horse, sheep, or pig) in a manner such that the mammal's BP is reduced after the administration. In some cases, aldosterone is not elevated in the mammal after the administration. In some cases, the mammal can be identified as having hypertension (e.g., RT).

Diuretics, commonly known as "water pills," are drugs that help the kidneys remove excess water and salt from the body, thus reducing the level of fluid in the blood vessels, which reduces blood pressure. Diuretics often are the first type of medication prescribed to patients presenting with high blood pressure. Diuretics that may be used to treat patients with high blood pressure include, without limitation, thiazide diuretics [e.g., chlorthalidone (Hygroton), chlorothiazide (Diuril), hydrochlorothiazide (Esidrix, Hydrodiuril, or Microzide), indapamide (Lozol), and metolazone (Mykrox, Zaroxolyn)], and others, such as amiloride (Midamor), bumetanide (Bumex), furosemide (Lasix), spironolactone (Aldactone), and triamterene (Dyrenium). Furosemide (abbreviated herein as "Fs") can increase the effectiveness of other antihypertensive agents, but Fs also activates the renin-angiotensin-aldosterone system.

Natriuretic polypeptides are polypeptides that can cause natriuresis - increased sodium excretion in the urine. Natriuretic polypeptides can be produced by the brain, heart, kidney, and/or vascular tissue. The natriuretic polypeptide family in humans includes the cardiac hormones atrial natriuretic peptide (ANP), B-type natriuretic peptide (BNP), C-type natriuretic peptide (CNP), and urodilatin (URO). Natriuretic polypeptides function via two well-characterized guanylyl cyclase receptors (NPR-A for ANP, BNP, and URO; and NPR-B for CNP) and the second messenger cyclic 3'5' guanosine monophosphate (cGMP) (Kuhn (2003) Circ. Res. 93:700-709; Tawaragi et al. (1991) Biochem. Biophys. Res. Commun. 175:645-651; and Komatsu et al. (1991) Endocrinol. 129:1104-1106). Natriuretic polypeptides can be effective to, for example, increase plasma cGMP levels, increase urinary cGMP excretion, increase net renal cGMP generation, increase urine flow, increase urinary sodium excretion, increase urinary potassium excretion, increase hematocrit, increase plasma BNP immunoreactivity, increase renal blood flow, increase plasma ANP immunoreactivity, decrease renal vascular resistance, decrease proximal and distal fractional reabsorption of sodium, decrease mean arterial pressure, decrease pulmonary wedge capillary pressure, decrease right atrial pressure, decrease pulmonary arterial pressure, decrease plasma renin activity, decrease plasma angiotensin II levels, decrease plasma aldosterone levels, decrease renal perfusion pressure, and/or decrease systemic vascular resistance.

MANP is a pGC-A/cGMP activator that can significantly lower blood pressure and vascular resistance. The methods provided herein can include, in part, treating a mammal with an MANP. As depicted in FIG. 1, MANP is an ANP-based peptide having an amino acid sequence that includes the 28 amino acid mature human ANP sequence (SLRRSSCFGGRMDRIGAQSGLGCNSFRY; SEQ ID NO:1) with an additional 12 amino acid carboxy terminus (RITAREDKQGWA; SEQ ID NO:2). The full length sequence of MANP is SLRRSSCFGGRMDRIGAQSGLGCNSFRYRIT AREDKQGWA (SEQ ID NO:3). A representative nucleic acid sequence encoding MANP is 5'-agcctgcggagatccagctgcttcgggggcaggatggacaggattggagcccagagcggactggg ctgtaacagcttccggtaccggataacagccagggaggacaagcagggctgggcctag-3' (SEQ ID NO:4).

As used herein, "an MANP" can have the amino acid sequence set forth in SEQ ID NO:3, or can be a variant of the sequence set forth in SEQ ID NO:3. Thus, in some cases, an MANP used in the methods provided herein can contain the entire amino acid sequence set forth in SEQ ID NO:3. In some cases, an MANP can contain the amino acid sequence set forth in SEQ ID NO:3, with the proviso that the amino acid sequence contains one or between one and ten (e.g., ten, between one and nine, between two and nine, between one and eight, between two and eight, between one and seven, between one and six, between one and five, between one and four, between one and three, two, or one) amino acid additions, subtractions, and/or substitutions. For example, an MANP can contain the amino acid sequence set forth in SEQ ID NO:3 with one, two, three, four, five, six, seven, eight, nine, or ten single amino acid residue additions, subtractions, or substitutions. In some cases, an MANP can have one or more additions, subtractions, and/or substitutions within the C-terminal portion of SEQ ID NO:3 (e.g., the last 12 amino acids of SEQ ID NO:3). Examples of such a polypeptide include, without limitation, a polypeptide having the amino acid sequence set forth in SEQ ID NO:3 where the threonine is deleted (SLRRSSCFGGRMDRIGAQSGLGCNSFRYRIA REDKQGWA; SEQ ID NO:5), the tryptophan is replaced with a tyrosine (SLRRSSCFGGRMDRIGAQSGLGCNSFRY RITAREDKQGYA; SEQ ID NO:6), a serine is added between the lysine and the glutamine (SLRRSSCFGGRMDRIGAQS GLGCNSFRYRITAREDKSQGWA; SEQ ID NO:7), or any combination thereof. In another example, an MANP can lack the last three residues of SEQ ID NO:3 (i.e., the glycine, tryptophan, and alanine residues of SEQ ID NO:3, as set forth in SEQ ID NO:8 (SLRRSSCFGGRMDRIGAQSGLGCNSFRY RITAREDKQ). In some cases, an MANP can have one or more additions, subtractions, and/or substitutions within the N-terminal portion of SEQ ID NO:3 (e.g., the first six amino acids of SEQ ID NO:3). Examples of such polypeptides include, without limitation, a polypeptide having the amino acid sequence set forth in SEQ ID NO:3 where four amino acids from urodilatin are added to the N-terminus (TAPRSLRRSSCFGGRMDRIGAQSGLGCNSFRYRITAREDKQGWA; SEQ ID NO:9), the arginine residues at positions 3 and 4 are substituted with lysine residues (SLKKSSCFGGRMD RIGAQSGLGCNSFRYRITAREDKQGWA; SEQ ID NO:10), the D-isoform of serine is substituted at the sixth position (SLRRSSCFGGRM DRIGAQSGLGCNSFRYRITA REDKQGWA; SEQ ID NO:11), the D-isoform of arginine is substituted at the fourth position and the serine at the fifth position is deleted (SLRRSCFGGRMDRIGAQSGL GCNSFRYRITAREDKQGWA; SEQ ID NO:12), threonine residues are substituted for the serine residues at positions 1, 5, and 6 (TLRRTTCFGGRMDRIGAQSGLGCNSFR YRITAREDKQGWA; SEQ ID NO:13), a tryptophan is substituted for the leucine at position 2 (SWRRSSCFGGRMDR IGAQSGLGCNSFRY RITAREDKQGWA; SEQ ID NO:14), or any combination thereof.

Any amino acid residue set forth in SEQ ID NO:3 can be subtracted, and any amino acid residue (e.g., any of the 20 conventional amino acid residues or any other type of amino acid such as ornithine or citrulline) can be added to the sequence set forth in SEQ ID NO:3. In some cases, an MANP can contain one or more chemical structures such as ε-aminohexanoic acid; hydroxylated amino acids such as 3-hydroxyproline, 4-hydroxyproline, (5R)-5-hydroxy-L-lysine, allo-hydroxylysine, and 5-hydroxy-L-norvaline; and/or glycosylated amino acids such as amino acids containing monosaccharides (e.g., D-glucose, D-galactose, D-mannose, D-glucosamine, and D-galactosamine) or combinations of monosaccharides.

MANPs having one or more amino acid additions, subtractions, or substitutions relative to the representative MANP sequence set forth in SEQ ID NO:3, also referred to herein as "variant" MANPs, can be generated using any suitable method. In some cases, amino acid substitutions can be made by selecting substitutions that do not differ significantly in their effect on maintaining (a) the structure of the peptide backbone in the area of the substitution, (b) the charge or hydrophobicity of the molecule at the target site, or (c) the bulk of the side chain. For example, naturally occurring residues can be divided into groups based on side-chain properties: (1) hydrophobic amino acids (norleucine, methionine, alanine, valine, leucine, and isoleucine); (2) neutral hydrophilic amino acids (cysteine, serine, and threonine); (3) acidic amino acids (aspartic acid and glutamic acid); (4) basic amino acids (asparagine, glutamine, histidine, lysine, and arginine); (5) amino acids that influence chain orientation (glycine and proline); and (6) aromatic amino acids (tryptophan, tyrosine, and phenylalanine). Substitutions made within these groups can be considered conservative substitutions. Non-limiting examples of useful conservative substitutions can include, without limitation, substitution of valine for alanine, lysine for arginine, glutamine for asparagine, glutamic acid for aspartic acid, serine for cysteine, asparagine for glutamine, aspartic acid for glutamic acid, proline for glycine, arginine for histidine, leucine for isoleucine, isoleucine for leucine, arginine for lysine, leucine for methionine, leucine for phenylalanine, glycine for proline, threonine for serine, serine for threonine, tyrosine for tryptophan, phenylalanine for tyrosine, and/or leucine for valine.

Further examples of conservative substitutions that can be made at any position within an MANP useful in the methods described herein are set forth in **TABLE 1.**

In some embodiments, an MANP can include one or more non-conservative substitutions. Non-conservative substitutions typically entail exchanging a member of one of the classes described above for a member of another class. Such production can be desirable to provide large quantities or alternative embodiments of such compounds. Whether an amino acid change results in a functional polypeptide can readily be determined by assaying the specific activity of the polypeptide variant using, for example, methods disclosed herein.

In some embodiments, an MANP can have a length of, for example, 35 to 45 amino acid residues (e.g., 35 to 40, 40 to 45, 35 to 37, 36 to 38, 37 to 39, 38 to 40, 39 to 41, 40 to 42, 41 to 43, 42 to 44, or 43 to 45 amino acid residues). In some embodiments, a natriuretic polypeptide can include an amino acid sequence as set forth in SEQ ID NO:3, but with a particular number of amino acid substitutions. For example, a natriuretic polypeptide can have the amino acid sequence of SEQ ID NO:3, but with one, two, three, four, or five amino acid substitutions. Examples of such amino acid sequences include, without limitation, MANP with a D-amino acid replacing one or more L-amino acids within the N-terminal region of the polypeptide (e.g., with a D-serine residue at position 6, as set forth in SEQ ID NO:1 1, or with a D-arginine at position 4, as set forth in SEQ ID NO: 12).

**TABLE 1**

| *Examples of conservative amino acid substitutions* | | |
|---|---|---|
| **Original Residue** | **Exemplary substitutions** | **Typical preferred substitutions** |
| Ala | Val, Leu, Ile | Val |
| Arg | Lys, Gln, Asn | Lys |
| Asn | Gln, His, Lys, Arg | Gln |
| Asp | Glu | Glu |
| Cys | Ser | Ser |
| Gln | Asn | Asn |
| Glu | Asp | Asp |
| Gly | Pro | Pro |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, Gln, Asn | Arg |
| Met | Leu, Phe, Ile | Leu |
| Phe | Leu, Val, Ile, Ala | Leu |
| Pro | Gly | Gly |
| Ser | Thr | Thr |
| Thr | Ser | Ser |
| Trp | Tyr | Tyr |
| Tyr | Trp, Phe, Thr, Ser | Phe |
| Val | Ile, Leu, Met, Phe, Ala, Norleucine | Leu |

In some embodiments, an MANP can include an amino acid sequence with at least 90% (e.g., at least 90%, at least 92.5%, at least 95%, at least 97.5%, or 100%) sequence identity to the reference sequence set forth in SEQ ID NO:3. Percent sequence identity is calculated by determining the number of matched positions in aligned amino acid sequences, dividing the number of matched positions by the total number of aligned amino acids, and multiplying by 100. A matched position refers to a position in which identical amino acids occur at the same position in aligned amino acid sequences. Percent sequence identity also can be determined for any nucleic acid sequence.

The percent sequence identity between a particular nucleic acid or amino acid sequence and a sequence referenced by a particular sequence identification number is determined as follows. First, a nucleic acid or amino acid sequence is compared to the sequence set forth in a particular sequence identification number using the BLAST 2 Sequences (Bl2seq) program from the stand-alone version of BLASTZ containing BLASTN version 2.0.14 and BLASTP version 2.0.14. This stand-alone version of BLASTZ can be obtained online at fr.com/blast or at ncbi.nlm.nih.gov. Instructions explaining how to use the Bl2seq program can be found in the readme file accompanying BLASTZ. Bl2seq performs a comparison between two sequences using either the BLASTN or BLASTP algorithm. BLASTN is used to compare nucleic acid sequences, while BLASTP is used to compare amino acid sequences. To compare two nucleic acid sequences, the options are set as follows: -i is set to a file containing the first nucleic acid sequence to be compared (e.g., C:\seq1.txt); -j is set to a file containing the second nucleic acid sequence to be compared (e.g., C:\seq2.txt); -p is set to blastn; -o is set to any desired file name (e.g., C:\output.txt); -q is set to -1; -r is set to 2; and all other options are left at their default setting. For example, the following command can be used to generate an output file containing a comparison between two sequences: C:\Bl2seq -i c:\seq1.txt -j c:\seq2.txt -p blastn -o c:\output.txt -q -1 -r 2. To compare two amino acid sequences, the options of Bl2seq are set as follows: -i is set to a file containing the first amino acid sequence to be compared (e.g., C:\seq1.txt); -j is set to a file containing the second amino acid sequence to be compared (e.g., C:\seq2.txt); -p is set to blastp; -o is set to any desired file name (e.g., C:\output.txt); and all other options are left at their default setting. For example, the following command can be used to generate an output file containing a comparison between two amino acid sequences: C:\Bl2seq -i c:\seq1.txt -j c:\seq2.txt -p blastp -o c:\output.txt. If the two compared sequences share homology, then the designated output file will present those regions of homology as aligned sequences. If the two compared sequences do not share homology, then the designated output file will not present aligned sequences.

Once aligned, the number of matches is determined by counting the number of positions where an identical nucleotide or amino acid residue is presented in both sequences. The percent sequence identity is determined by dividing the number of matches either by the length of the sequence set forth in the identified sequence (e.g., SEQ ID NO:3), or by an articulated length (e.g., 20 consecutive nucleotides or amino acid residues from a sequence set forth in an identified sequence), followed by multiplying the resulting value by 100. For example, an amino acid sequence that has 37 matches when aligned with the sequence set forth in SEQ ID NO:3 is 92.5 percent identical to the sequence set forth in SEQ ID NO:3 (i.e., 37 ÷ 40 x 100 = 92.5). It is noted that the percent sequence identity value is rounded to the nearest tenth. For example, 75.11, 75.12, 75.13, and 75.14 is rounded down to 75.1, while 75.15, 75.16, 75.17, 75.18, and 75.19 is rounded up to 75.2. It also is noted that the length value will always be an integer.

Isolated MANPs can be produced using any suitable method, including solid phase synthesis, and can be generated using manual techniques or automated techniques (e.g., using an Applied BioSystems (Foster City, CA) Peptide Synthesizer or a Biosearch Inc. (San Rafael, CA) automatic peptide synthesizer). Disulfide bonds between cysteine residues can be introduced by mild oxidation of the linear polypeptides using KCN as taught, e.g., in U.S. Patent No. 4,757,048. Natriuretic polypeptides also can be produced recombinantly, or obtained commercially.

The MANPs described herein typically are cyclic due to disulfide bonds between the cysteine residues (*see,* FIG. 1). In some embodiments, a sulfhydryl group on a cysteine residue can be replaced with an alternative group (e.g., -CH₂CH₂-). To replace a sulfhydryl group with a -CH₂- group, for example, a cysteine residue can be replaced by alpha-aminobutyric acid. Such cyclic analog polypeptides can be generated, for example, in accordance with the methodology of Lebl and Hruby ((1984) Tetrahedron Lett. 25:2067-2068), or by employing the procedure disclosed in U.S. Patent No. 4,161,521.

In addition, ester bridges can be formed by reacting the OH of serine or threonine with the carboxyl group of aspartic acid or glutamic acid to yield a bridge having the structure -CH₂CO₂CH₂-. Similarly, an amide can be obtained by reacting the side chain of lysine with aspartic acid or glutamic acid to yield a bridge having the structure - CH₂C(O)NH(CH)₄-. Methods for synthesis of these bridges are known in the art *(see,* e.g., Schiller et al. (1985) Biochem. Biophys. Res. Comm. 127:558, and Schiller et al. (1985) Int. J. Peptide Protein Res. 25:171). For example, one method for preparing esters of the present polypeptides, when using the Merrifield synthesis technique, is to cleave the completed polypeptide from the resin in the presence of the desired alcohol under either basic or acidic conditions, depending upon the resin. The C-terminal end of the polypeptide then can be directly esterified when freed from the resin, without isolation of the free acid. Amides of polypeptides also can be prepared using techniques (e.g., those known in the art) for converting a carboxylic acid group or precursor to an amide. One method for amide formation at the C-terminal carboxyl group includes cleaving the polypeptide from a solid support with an appropriate amine, or cleaving in the presence of an alcohol, yielding an ester, followed by aminolysis with the desired amine. Other bridge-forming amino acid residues and reactions are provided in, for example, U.S. Pat. No. 4,935,492. Preparation of peptide analogs that include non-peptidyl bonds to link amino acid residues also are known in the art. *See,* e.g., Spatola et al. (1986) Life Sci. 38:1243; Spatola (1983) Vega Data 1(3); Morley (1980) Trends Pharm. Sci. 463-468; Hudson et al. (1979) Int. J. Pept. Prot. Res. 14:177; Spatola, in Chemistry and Biochemistry of Amino Acid Peptides and Proteins, B. Weinstein, ed., Marcel Dekker, New York, p. 267 (1983); Hann (1982) J. Chem. Soc. Perkin Trans. 1:307; Almquist et al. (1980) J. Med. Chem. 23:1392; Jennings-White et al. (1982) Tetrahedron Lett. 23:2533; European Patent Application EP 45665; Holladay et al. (1983) Tetrahedron Lett. 24:4401; and Hruby (1982) Life Sci. 31:189.

N-acyl derivatives of an amino group of a polypeptide can be prepared by utilizing an N-acyl protected amino acid for the final condensation, or by acylating a protected or unprotected peptide. O-acyl derivatives can be prepared for example, by acylation of a free hydroxy peptide or peptide resin. Either acylation may be carried out using standard acylating reagents such as acyl halides, anhydrides, acyl imidazoles, and the like. Both N- and O-acylation may be carried out together, if desired.

In some cases, an MANP can be pegylated, acetylated, or both. In some cases, a polypeptide can be covalently attached to oligomers, such as short, amphiphilic oligomers that enable administration or improve the pharmacokinetic or pharmacodynamic profile of the conjugated polypeptide. The oligomers can comprise water soluble polyethylene glycol (PEG) and/or lipid soluble alkyls (short, medium, or long chain fatty acid polymers, such as, without limitation, palmitic acid, myristic acid, lauric acid, capric acid, or steric acid). The fatty acid molecule can be attached to the free amino terminus or to any lysine side chain (an epsilon amino group), and a lysine residue for this attachment can be placed at either the C-terminal or N-terminal end of the peptide. Linkage to PEG or another suitable polymer, or fusion to albumin or another suitable polypeptide can result in a modified natriuretic polypeptide having an increased half-life as compared to an unmodified natriuretic polypeptide. Without being bound by a particular mechanism, an increased serum half-life can result from reduced proteolytic degradation, immune recognition, or cell scavenging of the modified natriuretic polypeptide. Methods for modifying a polypeptide by linkage to PEG (also referred to as "PEGylation") or other polymers are known in the art, and include those set forth in U.S. Patent No. 6,884,780; PCT Publication No. WO 2004/047871; Cataliotti et al. ((2007) Trends Cardiovasc. Med. 17:10-14; Veronese and Mero (2008) BioDfugs 22:315-329; Miller et al. (2006) Bioconjugate Chem. 17:267-274; and Veronese and Pasut (2005) Drug Discov. Today 10:1451-1458, all of which are incorporated herein by reference in their entirety. Methods for modifying a polypeptide by fusion to albumin also are known in the art, and include those set forth in U.S. Patent Publication No. 20040086976, and Wang et al. (2004) Pharm. Res. 21:2105-2111, both of which are incorporated herein by reference in their entirety.

In some cases, an MANP can be fused to the Fc domain of an immunoglobulin molecule (e.g., an IgG1 molecule) such that active transport of the fusion polypeptide across epithelial cell barriers occurs via the Fc receptor. In some cases, a polypeptide can be a cyclic polypeptide. A cyclic polypeptide can be obtained by bonding cysteine residues, however, the replacement of a sulfhydryl group on the cysteine residue with an alternative group also is envisioned, for example, -CH₂-CH₂-. For example, to replace sulfhydryl groups with a --CH₂-- group, the cysteine residues can be replaced by the analogous alpha-aminobutyric acid. These cyclic analog peptides can be formed, for example, in accordance with the methodology of Lebl and Hruby (Tetrahedron Lett., 25:2067-2068, 1984), or by employing the procedure disclosed in U.S. Patent No. 4,161,521.

Salts of carboxyl groups of MANPs can be prepared by contacting a polypeptide with one or more equivalents of a desired base such as, for example, a metallic hydroxide base (e.g., sodium hydroxide), a metal carbonate or bicarbonate base (e.g., sodium carbonate or sodium bicarbonate), or an amine base (e.g., triethylamine, triethanolamine, and the like). Acid addition salts of polypeptides can be prepared by contacting the polypeptide with one or more equivalents of an inorganic or organic acid (e.g., hydrochloric acid).

The term "polypeptide" as used herein refers to a compound of two or more subunit amino acids, regardless of post-translational modification (e.g., phosphorylation or glycosylation). The subunits may be linked by peptide bonds or other bonds such as, for example, ester or ether bonds. The term "amino acid" refers to either natural and/or unnatural or synthetic amino acids, including D/L optical isomers.

The term "isolated" as used herein with reference to a polypeptide means that the polypeptide (1) is not associated with proteins found in nature, (2) is free of other proteins from the same source (e.g., free of human proteins), (3) is expressed by a cell from a different species, or (4) does not occur in nature. An isolated polypeptide can be, for example, encoded by DNA or RNA, including synthetic DNA or RNA, or some combination thereof.

The term "substantially pure" as used herein with reference to a polypeptide means the polypeptide is substantially free of other polypeptides, lipids, carbohydrates, and nucleic acid with which it is naturally associated. A substantially pure polypeptide can be any polypeptide that is removed from its natural environment and is at least 60 percent pure. A substantially pure polypeptide can be at least about 65, 70, 75, 80, 85, 90, 95, or 99 percent pure, or about 65 to 75, 75 to 80, 80 to 85, 85 to 90, 90 to 95, or 95 to 99 percent pure. Typically, a substantially pure polypeptide will yield a single major band on a non-reducing polyacrylamide gel. In some embodiments, a substantially pure polypeptide can be a chemically synthesized polypeptide.

Any method can be used to obtain a substantially pure polypeptide. For example, common polypeptide purification techniques such as affinity chromatography and HPLC as well as polypeptide synthesis techniques can be used. In addition, any material can be used as a source to obtain a substantially pure polypeptide. For example, tissue from wild-type or transgenic animals can be used as a source material. In addition, tissue culture cells engineered to over-express a particular polypeptide can be used to obtain substantially pure polypeptide. Further, a polypeptide can be engineered to contain an amino acid sequence that allows the polypeptide to be captured onto an affinity matrix. For example, a tag such as c-myc, hemagglutinin, polyhistidine, or Flag^{™} tag (Kodak) can be used to aid polypeptide purification. Such tags can be inserted anywhere within the polypeptide including at either the carboxyl or amino termini, or in between. Other fusions that can be used include enzymes that aid in the detection of the polypeptide, such as alkaline phosphatase.

MANPs (e.g., variant MANPs having conservative and/or non-conservative substitutions with respect to SEQ ID NO:3), as well as fragments of SEQ ID NO:3 or variant MANPs (e.g., fragments of any of SEQ ID NOS:3 to 14), can be screened for biological activity using any of a number of assays. For example, the activity of an MANP can be evaluated *in vitro* by testing its effect on cGMP production in cultured cells (e.g., cultured cardiac fibroblasts, aortic endothelial cells, or glomerular cells). Cells can be exposed to an MANP (e.g., an MANP at 10⁻¹⁰ to 10⁻⁴ M), and samples can be assayed to evaluate the polypeptide's effects on cGMP generation. cGMP generation can be detected and measured using, for example, a competitive RIA cGMP kit (Perkin-Elmer, Boston, MA).

The activity of an MANP also can be evaluated *in vivo* by, for example, testing its effects on factors such as plasma cGMP levels, urinary cGMP excretion, net renal generation of cGMP, glomerular filtration rate, blood pressure, heart rate, hemodynamic function such as cardiac output, pulmonary wedge pressure, systemic vascular resistance, and renal function such as renal blood flow, urine volume, and sodium excretion rate after administration to a mammal (e.g., a human, non-human primate, rodent, dog, cat, pig, sheep, horse, or cow). In some cases, such parameters can be evaluated after inducing hypertension in the mammal.

The term "nucleic acid" as used herein encompasses both RNA and DNA, including cDNA, genomic DNA, and synthetic (e.g., chemically synthesized) DNA. The nucleic acid can be double-stranded or single-stranded. Where single-stranded, the nucleic acid can be the sense strand or the antisense strand. In addition, nucleic acid can be circular or linear.

The term "isolated" as used herein with reference to nucleic acid refers to a naturally-occurring nucleic acid that is not immediately contiguous with both of the sequences with which it is immediately contiguous (one on the 5' end and one on the 3' end) in the naturally-occurring genome of the organism from which it is derived. For example, an isolated nucleic acid can be, without limitation, a recombinant DNA molecule of any length, provided one of the nucleic acid sequences normally found immediately flanking that recombinant DNA molecule in a naturally-occurring genome is removed or absent. Thus, an isolated nucleic acid includes, without limitation, a recombinant DNA that exists as a separate molecule (e.g., a cDNA or a genomic DNA fragment produced by PCR or restriction endonuclease treatment) independent of other sequences as well as recombinant DNA that is incorporated into a vector, an autonomously replicating plasmid, a virus (e.g., a retrovirus, adenovirus, or herpes virus), or into the genomic DNA of a prokaryote or eukaryote. In addition, an isolated nucleic acid can include a recombinant DNA molecule that is part of a hybrid or fusion nucleic acid sequence.

The term "isolated" as used herein with reference to nucleic acid also includes any non-naturally-occurring nucleic acid since non-naturally-occurring nucleic acid sequences are not found in nature and do not have immediately contiguous sequences in a naturally-occurring genome. For example, non-naturally-occurring nucleic acid such as an engineered nucleic acid is considered to be isolated nucleic acid. Engineered nucleic acid can be made using common molecular cloning or chemical nucleic acid synthesis techniques. Isolated non-naturally-occurring nucleic acid can be independent of other sequences, or incorporated into a vector, an autonomously replicating plasmid, a virus (e.g., a retrovirus, adenovirus, or herpes virus), or the genomic DNA of a prokaryote or eukaryote. In addition, a non-naturally-occurring nucleic acid can include a nucleic acid molecule that is part of a hybrid or fusion nucleic acid sequence.

It will be apparent to those of skill in the art that a nucleic acid existing among hundreds to millions of other nucleic acid molecules within, for example, cDNA or genomic libraries, or gel slices containing a genomic DNA restriction digest is not to be considered an isolated nucleic acid.

In some cases, an isolated nucleic acid molecule can be at least about 12 bases in length (e.g., at least about 13, 14, 15, 16, 17, 18, 19, 20, 25, 30, 40, 50, 60, 100, 120, 130, 140, 150, 250, 500, 750, 1000, 1500, 2000, 3000, 4000, or 5000 bases in length) and can hybridize, under hybridization conditions, to the sense or antisense strand of a nucleic acid having a sequence that encodes an MANP (e.g., an MANP having the sequence set forth in SEQ ID NO:3, or a variant thereof). The hybridization conditions can be moderately or highly stringent hybridization conditions.

For the purpose of this document, moderately stringent hybridization conditions mean the hybridization is performed at about 42°C in a hybridization solution containing 25 mM KPO₄ (pH 7.4), 5X SSC, 5X Denhart's solution, 50 µg/mL denatured, sonicated salmon sperm DNA, 50% formamide, 10% Dextran sulfate, and 1-15 ng/mL probe (about 5x10⁷ cpm/µg), while the washes are performed at about 50°C with a wash solution containing 2X SSC and 0.1% sodium dodecyl sulfate.

Highly stringent hybridization conditions mean the hybridization is performed at about 42°C in a hybridization solution containing 25 mM KPO₄ (pH 7.4), 5X SSC, 5X Denhart's solution, 50 µg/mL denatured, sonicated salmon sperm DNA, 50% formamide, 10% Dextran sulfate, and 1-15 ng/mL probe (about 5x10⁷ cpm/µg), while the washes are performed at about 65°C with a wash solution containing 0.2X SSC and 0.1% sodium dodecyl sulfate.

Isolated nucleic acid molecules encoding MANPs can be produced using standard techniques, including, without limitation, common molecular cloning and chemical nucleic acid synthesis techniques. For example, polymerase chain reaction (PCR) techniques can be used to obtain an isolated nucleic acid containing nucleotide sequence that encodes a natriuretic polypeptide as provided herein. PCR refers to a procedure or technique in which target nucleic acids are enzymatically amplified. Sequence information from the ends of the region of interest or beyond typically is employed to design oligonucleotide primers that are identical in sequence to opposite strands of the template to be amplified. PCR can be used to amplify specific sequences from DNA as well as RNA, including sequences from total genomic DNA or total cellular RNA. Primers typically are 14 to 40 nucleotides in length, but can range from 10 nucleotides to hundreds of nucleotides in length. General PCR techniques are described, for example in PCR Primer: A Laboratory Manual, ed. by Dieffenbach and Dveksler, Cold Spring Harbor Laboratory Press, 1995. When using RNA as a source of template, reverse transcriptase can be used to synthesize complementary DNA (cDNA) strands. Ligase chain reaction, strand displacement amplification, self-sustained sequence replication, or nucleic acid sequence-based amplification also can be used to obtain isolated nucleic acids. *See,* for example, Lewis (1992) Genetic Engineering News 12:1; Guatelli et al. (1990) Proc. Natl. Acad. Sci. USA 87:1874-1878; and Weiss (1991) Science 254:1292.

Isolated nucleic acids encoding MANPs also can be chemically synthesized, either as a single nucleic acid molecule (e.g., using automated DNA synthesis in the 3' to 5' direction using phosphoramidite technology) or as a series of oligonucleotides. For example, one or more pairs of long oligonucleotides (e.g., >100 nucleotides) can be synthesized that contain the desired sequence, with each pair containing a short segment of complementarity (e.g., about 15 nucleotides) such that a duplex is formed when the oligonucleotide pair is annealed. DNA polymerase is used to extend the oligonucleotides, resulting in a single, double-stranded nucleic acid molecule per oligonucleotide pair, which then can be ligated into a vector.

In addition, isolated nucleic acids encoding MANPs can be obtained by mutagenesis. For example, a reference sequence can be mutated using standard techniques including oligonucleotide-directed mutagenesis and site-directed mutagenesis through PCR. *See,* Short Protocols in Molecular Biology, Chapter 8, Green Publishing Associates and John Wiley & Sons, edited by Ausubel et al., 1992. Non-limiting examples of variant natriuretic polypeptides are provided herein.

Vectors containing nucleic acids such as those described herein also are provided. A "vector" is a replicon, such as a plasmid, phage, or cosmid, into which another DNA segment may be inserted so as to bring about the replication of the inserted segment. An "expression vector" is a vector that includes one or more expression control sequences, and an "expression control sequence" is a DNA sequence that controls and regulates the transcription and/or translation of another DNA sequence.

In an expression vector, a nucleic acid (e.g., a nucleic acid encoding a natriuretic polypeptide) can be operably linked to one or more expression control sequences. As used herein, "operably linked" means incorporated into a genetic construct so that expression control sequences effectively control expression of a coding sequence of interest. Examples of expression control sequences include promoters, enhancers, and transcription terminating regions. A promoter is an expression control sequence composed of a region of a DNA molecule, typically within 100 to 500 nucleotides upstream of the point at which transcription starts (generally near the initiation site for RNA polymerase II). To bring a coding sequence under the control of a promoter, it is necessary to position the translation initiation site of the translational reading frame of the polypeptide between one and about fifty nucleotides downstream of the promoter. Enhancers provide expression specificity in terms of time, location, and level. Unlike promoters, enhancers can function when located at various distances from the transcription site. An enhancer also can be located downstream from the transcription initiation site. A coding sequence is "operably linked" and "under the control" of expression control sequences in a cell when RNA polymerase is able to transcribe the coding sequence into mRNA, which then can be translated into the protein encoded by the coding sequence. Expression vectors thus can be useful to produce antibodies as well as other multivalent molecules.

Suitable expression vectors include, without limitation, plasmids and viral vectors derived from, for example, bacteriophage, baculoviruses, tobacco mosaic virus, herpes viruses, cytomegalovirus, retroviruses, vaccinia viruses, adenoviruses, and adeno-associated viruses. Numerous vectors and expression systems are commercially available from such corporations as Novagen (Madison, WI), Clontech (Palo Alto, CA), Stratagene (La Jolla, CA), and Invitrogen/Life Technologies (Carlsbad, CA).

An expression vector can include a tag sequence designed to facilitate subsequent manipulation of the expressed nucleic acid sequence (e.g., purification or localization). Tag sequences, such as green fluorescent protein (GFP), glutathione S-transferase (GST), polyhistidine, c-myc, hemagglutinin, or Flag^{™} tag (Kodak, New Haven, CT) sequences typically are expressed as a fusion with the encoded polypeptide. Such tags can be inserted anywhere within the polypeptide including at either the carboxyl or amino terminus.

Host cells containing vectors also are provided. The term "host cell" is intended to include prokaryotic and eukaryotic cells into which a recombinant expression vector can be introduced (e.g., vector encoding an MANP). As used herein, "transformed" and "transfected" encompass the introduction of a nucleic acid molecule (e.g., a vector) into a cell by one of a number of techniques. Although not limited to a particular technique, a number of these techniques are well established within the art. Suitable methods for transforming and transfecting host cells can be found, for example, in Sambrook et al., Molecular Cloning: A Laboratory Manual (2nd edition), Cold Spring Harbor Laboratory, New York (1989). For example, calcium phosphate precipitation, electroporation, heat shock, lipofection, microinjection, and viral-mediated nucleic acid transfer can be used introduce nucleic acid into cells. In addition, naked DNA can be delivered directly to cells in vivo as described elsewhere (U.S. Patent Nos. 5,580,859 and 5,589,466). The host cells can express the encoded polypeptide, but it is noted that cells containing an isolated nucleic acid molecule provided herein are not required to express a polypeptide. The isolated nucleic acid molecule transformed into a host cell can be integrated into the genome of the cell or maintained in an episomal state. Thus, host cells can be stably or transiently transfected with a construct containing an isolated nucleic acid molecule provided herein.

Any appropriate method can be used to introduce an isolated nucleic acid molecule into a cell *in vivo* or *in vitro.* For example, calcium phosphate precipitation, electroporation, heat shock, lipofection, microinjection, and viral-mediated nucleic acid transfer are methods that can be used to introduce an isolated nucleic acid molecule into a cell. In addition, naked DNA can be delivered directly to cells *in vivo* as described elsewhere (e.g., U.S. Patent Nos. 5,580,859 and 5,589,466, and continuations thereof). Further, isolated nucleic acid molecules can be introduced into cells by generating transgenic animals.

Any appropriate method can be used to identify cells containing an isolated nucleic acid molecule that encodes an MANP. Such methods include, without limitation, PCR and nucleic acid hybridization techniques such as Northern and Southern analyses. In some cases, immunohistochemistry and biochemical techniques can be used to determine if a cell contains a particular isolated nucleic acid molecule by detecting the expression of a polypeptide encoded by that nucleic acid molecule.

Isolated MANPs, nucleic acids encoding MANPs, and diuretic agents as described herein can be used to treat mammals identified as having conditions such as hypertension (e.g., RH). Thus, one or more diuretic agents and one or more MANPs - or nucleic acids encoding one or more MANPs - can be incorporated into a composition for administration to a mammal (e.g., a mammal having, or at risk for having, hypertension, RH, and/or cardiorenal disease). Any suitable method can be used to formulate and subsequently administer a composition. Dosages typically are dependent on the responsiveness of the mammal to the agent(s) being administered, with the course of treatment lasting from several days to several months or longer, or until a suitable response is achieved. Persons of ordinary skill in the art routinely determine optimum dosages, dosing methodologies and repetition rates. Optimum dosages can vary depending on the relative potency of each agent (e.g., an MANP and Fs), and generally can be estimated based on the EC₅₀ found to be effective in *in vitro* and/or *in vivo* animal models. Compositions containing one or more MANPs and one or more diuretic agents as described herein can be given once or more daily, weekly, monthly, or even less often, or can be administered continuously for a period of time (e.g., hours, days, or weeks). For example, an MANP or a composition containing am MANP can be administered to a patient at a dose of at least about 0.01 ng MANP/kg to about 100 mg MANP/kg of body mass. In some cases, an MANP or a composition containing an MANP can be administered as an infusion for one to 30 days or longer (e.g., at a dose of about 1 pmol MANP/kg/minute to about 500 nmol MANP/kg/minute).

One or more MANPs or nucleic acids encoding one or more MANPs, and one or more diuretics can be admixed, encapsulated, conjugated or otherwise associated with each other and/or other molecules, molecular structures, or mixtures of compounds such as, for example, liposomes, receptor or cell targeted molecules, or oral, topical or other formulations for assisting in uptake, distribution and/or absorption.

In some embodiments, a composition can contain an MANP (or nucleic acid encoding the MANP), a diuretic agent, or both, in combination with a pharmaceutically acceptable carrier. Pharmaceutically acceptable carriers include, for example, pharmaceutically acceptable solvents, suspending agents, or any other pharmacologically inert vehicles for delivering polypeptides and/or other compounds to a subject. Pharmaceutically acceptable carriers can be liquid or solid, and can be selected with the planned manner of administration in mind so as to provide for the desired bulk, consistency, and other pertinent transport and chemical properties, when combined with one or more therapeutic compounds and any other components of a given pharmaceutical composition. Useful pharmaceutically acceptable carriers include, without limitation: water; saline solution; binding agents (e.g., polyvinylpyrrolidone or hydroxypropyl methylcellulose); fillers (e.g., lactose or dextrose and other sugars, gelatin, or calcium sulfate); lubricants (e.g., starch, polyethylene glycol, or sodium acetate); disintegrates (e.g., starch or sodium starch glycolate); and wetting agents (e.g., sodium lauryl sulfate).

An MANP or nucleic acid and a diuretic agent can be present within the same composition, or can be present in separate compositions that are administered at the same time or at different times, and are administered via the same route or via separate routes. Pharmaceutical compositions containing MANPs and diuretic agents as described herein can be administered by a number of methods, depending upon whether local or systemic treatment is desired. Administration can be, for example, oral or parenteral (e.g., by subcutaneous, intrathecal, intraventricular, intramuscular, or intraperitoneal injection, or by intravenous (i.v.) drip). Administration can be rapid (e.g., by injection) or can occur over a period of time (e.g., by slow infusion or administration of slow release formulations).

Compositions and formulations for parenteral, intrathecal or intraventricular administration include sterile aqueous solutions (e.g., sterile physiological saline), which also can contain buffers, diluents and other suitable additives (e.g., penetration enhancers, carrier compounds and other pharmaceutically acceptable carriers). Compositions and formulations for oral administration include, for example, powders or granules, suspensions or solutions in water or non-aqueous media, capsules, sachets, or tablets. Such compositions also can incorporate thickeners, flavoring agents, diluents, emulsifiers, dispersing aids, or binders.

Pharmaceutical compositions include, but are not limited to, solutions, emulsions, aqueous suspensions, and liposome-containing formulations. These compositions can be generated from a variety of components that include, for example, preformed liquids, self-emulsifying solids and self-emulsifying semisolids. Emulsion formulations are particularly useful for oral delivery of therapeutic compositions due to their ease of formulation and efficacy of solubilization, absorption, and bioavailability. Liposomes can be particularly useful due to their specificity and the duration of action they offer from the standpoint of drug delivery.

Compositions can contain any pharmaceutically acceptable salts, esters, or salts of such esters, or any other compound which, upon administration to a subject, is capable of providing (directly or indirectly) the biologically active metabolite or residue thereof for the relevant compound (e.g., an MANP or a diuretic agent). Accordingly, for example, this document provides pharmaceutically acceptable salts of MANPs and diuretics, prodrugs and pharmaceutically acceptable salts of such prodrugs, and other bioequivalents. A prodrug is a therapeutic agent that is prepared in an inactive form and is converted to an active form (i.e., drug) within the body or cells thereof by the action of endogenous enzymes or other chemicals and/or conditions. The term "pharmaceutically acceptable salts" refers to physiologically and pharmaceutically acceptable salts of the MANPs and diuretic agents useful in the methods provided herein (i.e., salts that retain the desired biological activity of the parent compounds without imparting undesired toxicological effects). Examples of pharmaceutically acceptable salts include, but are not limited to, salts formed with cations (e.g., sodium, potassium, calcium, or polyamines such as spermine); acid addition salts formed with inorganic acids (e.g., hydrochloric acid, hydrobromic acid, sulfuric acid, phosphoric acid, or nitric acid); salts formed with organic acids (e.g., acetic acid, citric acid, oxalic acid, palmitic acid, or fumaric acid); and salts formed with elemental anions (e.g., bromine, iodine, or chlorine).

Compositions additionally can contain other adjunct components conventionally found in pharmaceutical compositions. Thus, the compositions also can include compatible, pharmaceutically active materials such as, for example, antipruritics, astringents, local anesthetics or anti-inflammatory agents, or additional materials useful in physically formulating various dosage forms of the compositions, such as dyes, flavoring agents, preservatives, antioxidants, opacifiers, thickening agents, and stabilizers. Furthermore, the composition can be mixed with auxiliary agents, e.g., lubricants, preservatives, stabilizers, wetting agents, emulsifiers, salts for influencing osmotic pressure, buffers, colorings, flavorings, penetration enhancers, and aromatic substances. When added, however, such materials should not unduly interfere with the biological activities of the other components within the compositions.

In some cases, an MANP can be formulated as a sustained release dosage form. For example, an MANP or a diuretic agent can be formulated into a controlled release formulation. In some cases, coatings, envelopes, or protective matrices can be formulated to contain one or more of the MANPs described herein. Such coatings, envelopes, and protective matrices can be used to coat indwelling devices such as stents, catheters, and peritoneal dialysis tubing. In some cases, a polypeptide can be incorporated into polymeric substances, liposomes, microemulsions, microparticles, nanoparticles, or waxes.

Pharmaceutical formulations as disclosed herein, which can be presented conveniently in unit dosage form, can be prepared according to any suitable method, including conventional techniques known in the pharmaceutical industry. Such techniques include the step of bringing into association the active ingredient(s) with the desired pharmaceutical carrier(s). Typically, the formulations can be prepared by uniformly and intimately bringing the active ingredients into association with liquid carriers or finely divided solid carriers or both, and then, if necessary, shaping the product. Formulations can be sterilized if desired, provided that the method of sterilization does not interfere with the effectiveness of the molecules(s) contained in the formulation.

In some embodiments, an MANP and/or a diuretic agent such as Fs can be formulated for subcutaneous delivery via injection, depot polymers, drug patch, pump, or microparticle/nano particle. By way of example and not limitation, PCT Publication No. WO 2008/061355 discloses materials and methods for formulating a polypeptide for delivery in a hydrogel tube. The polypeptide can be mixed with one or more excipients that are pharmaceutically acceptable and are compatible with the polypeptide in amounts suitable for use in the methods described herein. For example, a polypeptide can be combined with one or more excipients such as, without limitation, microcrystalline cellulose, colloidal silicon dioxide, lactose, starch, sorbitol, cyclodextrin, and combinations thereof. The excipient can be a solid, semi-solid, or liquid material that acts as a vehicle, carrier, or medium for the polypeptide. In some embodiments, the polypeptide can be compressed, compacted, or extruded with one or more excipients prior to inserting it into a hydrogel tube. Such formulations can result in a pharmaceutical composition with desirable release properties, improved stability, and/or other desirable properties.

Pharmaceutical compositions also can include auxiliary agents or excipients, such as glidants, dissolution agents, surfactants, diluents, binders, disintegrants, and/or lubricants. For example, dissolution agents can increase the dissolution rate of a polypeptide from a dosage formulation, and can include, for example, organic acids and/or salts of organic acids (e.g., sodium citrate with citric acid). Other examples of excipients useful in such formulations include synthetic, semi-synthetic, modified, and natural polymers (e.g., lactose, dextrose, sucrose, trehalose, sorbitol, mannitol, starches, gum acacia, calcium silicate, microcrystalline cellulose, polyvinylpyrrolidone, cellulose, water, syrup, PEG, cyclodextrin, alkoxy- modified cyclodextrins, hydroxyethylcellulose, hydroxypropylcellulose, microcrystalline cellulose, albumin, dextran, malitol, xylitol, kaolin, and methyl cellulose). The polypeptide also can be mixed with a lubricating agent (e.g., talc, magnesium stearate, stearic acid, or mineral oil, calcium stearate, hydrogenated vegetable oils, sodium benzoate, sodium chloride, leucine carbowax, magnesium lauryl sulfate, or glyceryl monostearate), a wetting agent, an emulsifying and suspending agent, or a preserving agent (e.g., methyl or propyl hydroxybenzoate).

Other agents that can be added to a pharmaceutical composition can alter the pH of the microenvironment on dissolution and establishment of a therapeutically effective plasma concentration profile of an MANP or a diuretic compound. Such agents include salts of inorganic acids and magnesium hydroxide. Other agents that can be used include surfactants and other solubilizing materials.

Useful diluents include, for example, pharmaceutically acceptable inert fillers such as microcrystalline cellulose, lactose, sucrose, fructose, glucose dextrose, or other sugars, dibasic calcium phosphate, calcium sulfate, cellulose, ethylcellulose, cellulose derivatives, kaolin, mannitol, lactitol, maltitol, xylitol, sorbitol, or other sugar alcohols, dry starch, saccharides, dextrin, maltodextrin or other polysaccharides, inositol or combinations thereof. In some cases, water-soluble diluents can be particularly useful.

Glidants can be used to improve the flow and compressibility of composition ingredients during processing. Useful glidants include, for example, colloidal silicon dioxide (also referred to as colloidal silica, fumed silica, light anhydrous silicic acid, silicic anhydride, and silicon dioxide fumed).

Surfactants that are suitable for use in the pharmaceutical compositions described herein include, without limitation, sodium lauryl sulphate, polyethylene stearates, polyethylene sorbitan fatty acid esters, polyoxyethylene castor oil derivatives, polyoxyethylene alkyl ethers, benzyl benzoate, cetrimide, cetyl alcohol, docusate sodium,, glyceryl monooleate, glyceryl monostearate, glyceryl palmitostearate, lecithin, medium chain triglycerides, monoethanolamine, oleic acid, poloxarners, polyvinyl alcohol and sorbitan fatty acid esters.

Suitable disintegrants include, for example, starches, sodium starch glycolate, crospovidone, croscarmellose, microcrystalline cellulose, low substituted hydroxypropyl cellulose, pectins, potassium methacrylate- divinylbenzene copolymer, polyvinyl alcohol), thylamide, sodium bicarbonate, sodium carbonate, starch derivatives, dextrin, beta cyclodextrin, dextrin derivatives, magnesium oxide, clays, bentonite, and combinations thereof.

In some embodiments, an MANP and/or a diuretic agent can be incorporated into a hydrogel delivery system. For example, an MANP can be formulated for subcutaneous delivery to a patient via a xerogel-hydrogel system that can release the polypeptide in a continuous sustained manner over an extended period of time. *See,* for example, U.S. Patent No. 5,226,325, and PCT Publication No. WO 2004/071736.

Liquid polymerizable materials useful in the preparation of hydrogel tubes include a wide variety of polymerizable hydrophilic, and ethylenically unsaturated compounds. *See,* for example, the compounds listed in PCT Publication No. WO 2008/061355. Mixtures of such hydrophilic monomers typically are used in the polymerization reaction. The type and proportion of monomers are selected to yield a polymer (e.g., a crosslinked homogeneous polymer) that on hydration possesses the desired characteristics (e.g., equilibrium water content (EWC) value and/or pore size) for the contemplated application or use.

In some cases, the polymerization of hydrophilic monomeric mixtures can result in homogeneous hydrophilic copolymers which dissolve, to a varying extent, in an aqueous medium. In such cases, a small amount (e.g., up to about 3 percent) of a copolymerizable polyethylenically unsaturated crosslinking agent can be included in the monomeric mixture to obtain homogeneous crosslinked copolymers that are water-insoluble as well as water-swellable. A slightly crosslinked homopolymer of (hydroxyethyl)methacrylate (HEMA) has an EWC value of about 38%. Crosslinked copolymers of HEMA and N-(2-hydroxypropyl) methacrylamide (HPMA) have EWC values below 38%, while crosslinked copolymers of HEMA and acrylamide exhibit EWC values above 38 w/v %. Therefore, depending on the useful or effective elution rate of the polypeptide, copolymer hydrogels can be customized to elute the polypeptide at the desired rate. Typically, copolymers contain about 15 to about 70 weight % of HEMA units and from about 85 to 30 weight % of a second ethylenic monomer, and thus possess EWC values in the range of from about 20% to about 75%. In some embodiments, a mixture of copolymers can further contain a small amount of a polyethylenically unsaturated crosslinking agent [e.g., ethyleneglycol dimethacrylate ("EDMA") or trimethylolpropane trimethacrylate ("TMPTMA")].

In some embodiments, a pharmaceutical composition for controlled release delivery of an MANP in a subject can include (a) a complex of the polypeptide (where the polypeptide has at least one basic functional group) and a polyanion derived from hexahydroxycyclohexane (where the polyanion has at least two negatively charged functional groups); and (b) a pharmaceutically acceptable carrier containing a biodegradable, water-insoluble polymer. Such compositions are described in, for example, PCT Publication No. WO 2006/017852, and can be prepared in the form of solutions, suspensions, dispersions, emulsions, drops, aerosols, creams, semisolids, pastes, capsules, tablets, solid implants, or microparticles, for example. The term "controlled release delivery," as used herein, refers to continual delivery of a pharmaceutical agent *in vivo* over a period of time (e.g., several days to weeks or months) following administration. Sustained controlled release delivery of an MANP can be demonstrated by, for example, continued therapeutic effects of the polypeptide over time (e.g., continued reductions in symptoms over time). Sustained delivery of the polypeptide also can be demonstrated by detecting the presence of the polypeptide *in vivo* over time. The compositions can provide a low initial burst delivery, followed by stable, controlled release of the polypeptide *in vivo* for prolonged periods of time (e.g., from days to months).

In such embodiments, a physically and chemically stable complex can form upon appropriate combining of a polypeptide and a polyanion. The complex can take the form of a precipitate that is produced upon combining an aqueous preparation of the polypeptide and the polyanion. Optionally, one or more pharmaceutically acceptable excipients can be incorporated into the complex. Such excipients can function as stabilizers for the polypeptide and/or the complex. Non-limiting examples of suitable excipients include sodium bisulfite, p-aminobenzoic acid, thiourea, glycine, methionine, mannitol, sucrose, and PEG.

A stable complex between a polypeptide and a polyanion can be incorporated into a pharmaceutically acceptable carrier containing a biodegradable water-insoluble polymer, optionally with one or more excipients. The term "biodegradable water-insoluble polymer" refers to biocompatible and/or biodegradable synthetic and natural polymers that can be used *in vivo*. The term also is meant to include polymers that are insoluble or become insoluble in water or biological fluid at 37°C. The polymers can be purified (e.g., to remove monomers and oligomers) using techniques known in the art. *See,* e.g., U.S. Patent No. 4,728,721. Examples of useful polymers include, without limitation, polylactides, polyglycolides, poly(lactide-co- glycolide)s, polycaprolactones, polydioxanones, polycarbonates, polyhydroxybutyrates, polyalkylene oxalates, polyanhydrides, polyamides, polyesteramides, polyurethanes, polyacetals, polyorthocarbonates, polyphosphazenes, polyhydroxyvalerates, polyalkylene succinates, and polyorthoesters, and copolymers, block copolymers, branched copolymers, terpolymers, and combinations thereof.

Biodegradable water-insoluble polymers also can include end capped, end uncapped, or mixtures of end capped and end uncapped polymers. An end capped polymer generally is defined as having capped carboxyl end groups, while an uncapped polymer has free carboxyl end groups.

Factors to consider when determining suitable molecular weights for the polymer can include desired polymer degradation rate, mechanical strength, and rate of dissolution of polymer in solvent. Useful molecular weights for polymers can be from about 2,000 Daltons to about 150,000 Daltons, for example, with a polydispersity of from 1.1 to 2.8, depending upon which polymer is selected for use.

The pharmaceutically acceptable carrier can be a carrier with environment responsive properties (e.g., thermosensitive, pH sensitive, or electrical sensitive), in the form of an injectable solution or suspension, particle, film, pellet, cylinder, disc, microcapsule, microsphere, nanosphere, microparticle, wafer, micelle, liposome, or any other polymeric configuration useful for drug delivery.

Methods of forming various pharmaceutically acceptable polymer carriers include those that are known in the art. *See,* for example, U.S. Patent Nos. 6,410,044; 5,698,213; 6,312,679; 5,410,016; 5.529,914; 5,501,863; 4,938,763; 5,278,201; and 5,278,202; and PCT Publication No. WO 93/16687.

Compositions can be produced when a polypeptide/polyanion complex is dispersed in a polymeric matrix to form a solid implant, which can be injected or implanted into a subject. Such implants can be prepared using conventional polymer melt- processing techniques, such as extrusion, compression molding, and injection molding, for example. Preparations of such implants can be carried out under aseptic conditions, or alternatively by terminal sterilization by irradiation (e.g., using gamma irradiation or electron beam sterilization).

In some embodiments, compositions in the form of microspheres can be produced by encapsulating a polypeptide/polyanion complex in a polymeric carrier, using various biocompatible and/or biodegradable polymers having properties that are suitable for delivery to different biological environments or for effecting specific functions. The rate of dissolution and, therefore, delivery of polypeptide is determined by factors such as the encapsulation technique, polymer composition, polymer crosslinking, polymer thickness, polymer solubility, and size and solubility of polypeptide /polyanion complex.

To prepare such microspheres, a polypeptide/polyanion complex to be encapsulated can be suspended in a polymer solution in an organic solvent, such that the polymer solution completely coats the polypeptide/polyanion complex. The suspension then can be subjected to a microencapsulation technique such as spray drying, spray congealing, emulsion, or solvent evaporation emulsion. For example, the suspended complexes or microparticles along with the polymer in an organic solvent can be transferred to a larger volume of an aqueous solution containing an emulsifier, such that the organic solvent evaporates or diffuses away from the polymer and the solidified polymer encapsulates the polypeptide/polyanion complex.

Emulsifiers useful to prepare encapsulated polypeptide/polyanion complexes include poloxamers and polyvinyl alcohol, for example. Organic solvents useful in such methods include acetic acid, acetone, methylene chloride, ethyl acetate, chloroform, and other non-toxic solvents that will depend on the properties of the polymer. Solvents typically are chosen that solubilize the polymer and are ultimately non-toxic.

In some embodiments, an MANP can be formulated in a depot, which can provide constantly high exposure levels and may reach high exposure levels rapidly (with a short or no lag phase). *See,* e.g., U.S. Publication No. 2010/0266704. Depot formulations can include an MANP or a pharmaceutically-acceptable salt thereof (e.g., an acid addition salt with an inorganic acid, polymeric acid, or organic acid). Acid addition salts can exist as mono- or divalent salts, depending on whether one or two acid equivalents are added.

As described in U.S. Publication No. 2010/0266704, depot formulations can contain two different linear poly(lactic-co-glycolic acid) (PLGA) polymers having a molar ratio of lactide:glycolide comonomer (L:G) from 85:15 to 65:35, where at least one of the polymers has a low inherent viscosity. Such formulations can provide sustained high plasma levels of the polypeptide for extended periods of time. Examples of suitable polymers include the linear poly(D,L-lactide) and poly(D,L-lactide-co-glycolide) polymers sold under the trade names RESOMER^{®}, LACTEL^{®}, and MEDISORB^{®} by Boehringer Ingelheim Pharma GmBH & Co. KG (Ingelheim, Germany), Absorbable Polymers International (Pelham, AL), and Alkermes, Inc. (Cambridge, MA), respectively.

High exposure depot formulations for subcutaneous administration can show immediate or at least very rapid action, such that therapeutic plasma concentrations are achieved in a short time (e.g., one, two, three, four, five, six, or seven days after subcutaneous injection), and can show constantly high exposure levels over about one month or longer.

In some embodiments, the depot formulations provide herein can contain two different PLGA polymers mixed or blended in a % wt ratio of 95:5 to 50:50 (e.g., 85:15 to 50:50, 80:20 to 60:40, 90:10, 85:15, 80:20, 75:25, 70:30, 65:35, 60:40, 55:45 or 50:50% wt). In some embodiments, the polymer with the higher inherent viscosity can have a higher % wt than the polymer with the lower inherent viscosity. In some embodiments, the polymer with the higher inherent viscosity can have an ester endgroup. Depot formulations can contain further polymers, including other linear or star shaped PLGA polymers, or poly(D,L-lactide-co-glycolide) (PLG) or polylactic acid (PLA) polymers, provided that favorable PK properties are retained.

The polypeptide content of the depot formulation (the loading) can be in a range of 1% to 30% (e.g., 10% to 25%, more preferred 15% to 20%. The loading is defined as the weight ratio of polypeptide to the total mass of the PLGA formulation.

Depot compositions can be manufactured aseptically, or can be manufactured non-aseptically and terminally sterilized (e.g., using gamma irradiation). Terminal sterilization can result in a product with the highest sterility assurance possible.

Depot compositions also can contain one or more pharmaceutical excipients that can modulate the release behavior of the polypeptide. Such excipients can be present in the composition in an amount of about 0.1% to about 50%. Suitable excipients include, without limitation, polyvinyl alcohol, polyvinyl pyrrolidone, carboxymethyl cellulose sodium, dextrin, PEG, surfactants such as poloxamers (also known as poly(oxyethylene-block-oxypropylene), poly(oxyethylene)-sorbitan-fatty acid esters commercially available under the trade name TWEEN^{®}, sorbitan fatty acid esters, lecithins, inorganic salts such as zinc carbonate, magnesium hydroxide, magnesium carbonate, protamine, and natural or synthetic polymers bearing amine-residues such as polylysine.

Depot compositions can contain a mixture or blend of different polymers in terms of compositions, molecular weight and/or polymer architectures. A polymer blend is defined herein as a solid solution or suspension of two different linear polymers in one implant or microparticle. A mixture of depots is defined herein as a mixture of two depot-like implants or microparticles or semisolid formulations of different composition with one or more PLGAs in each depot. Pharmaceutical depot compositions in which two PLGAs are present as a polymer blend can be particularly useful.

Pharmaceutical depot compositions can be in the form of implants, semisolids (gels), liquid solutions, microparticles, or suspensions that solidify *in situ* once they are injected. The following paragraphs are focused on polymer microparticles, although the descriptions also are applicable for implants, semisolids, and liquids.

Microparticles can have a diameter from a few submicrons to a few millimeters (e.g., from about 0.01 micron to about 2 mm, about 0.1 micron to about 500 microns, about 10 to about 200 microns, about 10 to about 130 microns, or about 10 to about 90 microns).

In some embodiments, microparticles can be mixed or coated with an anti-agglomerating agent. Suitable anti-agglomerating agents include, for example, mannitol, glucose, dextrose, sucrose, sodium chloride, and water soluble polymers such as polyvinyl alcohol, polyvinyl pyrrolidone and PEG.

Microparticles can be manufactured using processes known in the art, such as coacervation or phase separation, spray drying, or water-in-oil (W/O), water-in-oil-in-water (W/O/W), or solids-in-oil-in-water (S/O/W) emulsion/suspension methods followed by solvent extraction or solvent evaporation. Emulsion/suspension methods can be particularly useful, and can include the following steps:
(i) preparing an internal organic phase, comprising
   (a) dissolving a polymer or polymers in a suitable organic solvent (e.g., ethyl acetate, acetone, THF, acetonitrile, or a halogenated hydrocarbon such as methylene chloride, chloroform, or hexafluoroisopropanol) or solvent mixture, and optionally dissolving/dispersing suitable additives;
   (b) dissolving/suspending/emulsifying a polypeptide in the polymer solution obtained in step (a);
(ii) preparing an external aqueous phase containing one or more stabilizers (e.g., poly(vinylalcohol), hydroxyethyl cellulose, hydroxypropyl cellulose, poly(vinyl pyrolidone), or gelatin) and optionally a buffer salt;
(iii) mixing the internal organic phase with the external aqueous phase to form an emulsion; and
(iv) hardening the microparticles by solvent evaporation or solvent extraction, washing the microparticles (e.g., with water), collecting and drying the microparticles (e.g., by freeze-drying or drying under vacuum), and sieving the microparticles (e.g., through 140 µm).

A dry microparticle composition can be terminally sterilized by gamma irradiation, either in bulk or after dispensing into the final container. In some embodiments, bulk sterilized microparticles can be resuspended in a suitable vehicle and dispensed into a suitable device such as double chamber syringe with subsequent freeze drying.

In some embodiments, microparticle depot compositions can include a vehicle to facilitate reconstitution. In addition, prior to administration, microparticles can be suspended in a suitable vehicle for injection (e.g., a water-based vehicle containing one or more pharmaceutical excipients such as mannitol, sodium chloride, glucose, dextrose, sucrose, or glycerin, and/or one or more non-ionic surfactants such as a poloxamer, poly(oxyethylene)-sorbitan-fatty acid ester, carboxymethyl cellulose sodium, sorbitol, poly(vinylpyrrolidone), or aluminium monostearate).

This document also provides methods for treating a mammal having a cardiovascular disorder (e.g., hypertension, resistant hypertension, or myocardial infarction). The methods can include administering (e.g., oral, parenteral, or a combination of oral and parenteral methods) an MANP and a diuretic agent as provided herein. The treatment can beneficially affect or alleviate one or more symptoms associated with the disorder, or one or more underlying causes of the disorder. For example, the treatment can reduce blood pressure in the mammal.

Before administering an MANP or nucleic acid and a diuretic agent to a mammal, or one or more compositions containing an MANP and a diuretic agent, a mammal can be assessed to determine whether or not the mammal has a need for treatment of a cardiovascular disorder such as hypertension (e.g., RH). After identifying the mammal as being in need for such treatment, the mammal can be treated with a composition as described herein. For example, a composition containing an MANP can be administered to the mammal in any amount, at any frequency, and for any duration effective to achieve a desired outcome (e.g., to reduce blood pressure in the mammal, or to prevent or delay further elevation of blood pressure in the mammal).

In some embodiments, a composition containing an MANP can be administered parenterally (e.g., by injection, such as subcutaneous injection, or by oral ingestion) at a dose of about 0.01 ng polypeptide/kg to about 100 mg polypeptide/kg of body mass (e.g., about 10 ng polypeptide/kg to about 50 mg polypeptide/kg, about 20 ng polypeptide/kg to about 10 mg polypeptide/kg, about 0.1 ng polypeptide/kg to about 20 ng polypeptide/kg, about 3 ng polypeptide/kg to about 10 ng polypeptide/kg, about 10 ng/polypeptide/kg to about 50 ng polypeptide/kg, about 50 ng polypeptide/kg to about 100 µg polypeptide/kg, about 1 µg polypeptide/kg to about 10 µg polypeptide/kg, about 1 µg polypeptide/kg to about 5 µg polypeptide/kg, about 3 µg polypeptide/kg to about 7 µg polypeptide/kg, about 5 µg polypeptide/kg to about 10 µg polypeptide/kg, about 10 µg polypeptide/kg to about 20 µg polypeptide/kg, or about 20 µg polypeptide/kg to about 100 µg polypeptide/kg) of body mass, although other dosages also may provide beneficial results. In some cases, an MANP can be administered by infusion at a dose of, for example, about 1 pmol/kg/minute to about 500 nmol/kg/minute (e.g., about 1 to 10 pmol/kg/minute, about 10 to 100 pmol/kg/minute, about 100 to 300 pmol/kg/minute, about 250 to 500 pmol/ kg/minute, about 500 pmol/kg/minute to about 1 nmol/kg/minute, about 1 to 10 nmol/kg/minute, about 10 to 100 nmol/kg/minute, about 100 to 300 nmol/kg/minute, or about 250 to 500 nmol/kg/minute).

A diuretic agent can be administered at a dose of at least about 1 µg/kg to about 500 mg/kg of body mass (e.g., about 1 to 10 µg/kg, about 10 to 50 µg/kg, about 50 to 100 µg/kg, about 100 to 500 µg/kg, about 500 µg/kg to about 1 mg/kg, about 1 to 5 mg/kg, about 5 to 10 mg/kg, about 10 to 50 mg/kg, about 50 to 100 mg/kg, or about 100 to 500 mg/kg) of body mass, although other dosages also may provide beneficial results. It is noted that the dosage of diuretic can be decreased over time, given the potentiation effect of the MANP. Thus, while a diuretic may initially be administered at a dose of about 1 to 5 mg/kg, the dose may be titrated down as time passes.

An MANP and a diuretic agent can be administered once (e.g., by implantation or injection of a depot composition), or more than once (e.g., by repeated injections or oral administrations). When administered more than once, the frequency of administration can range from one or more times a day (e.g., one, two, three, four, or more times a day) to about once every other month (e.g., three to five times a week, about once a week, about twice a month, about once a month, or about once every other month). In addition, the frequency of administration can remain constant or can be variable during the duration of treatment. Various factors can influence the actual frequency of administration used for a particular application. For example, the effective amount, duration of treatment, route of administration, and severity of condition may require an increase or decrease in administration frequency.

In some embodiments, an MANP natriuretic polypeptide or a composition containing an MANP can be administered via a first route (e.g., intravenously) for a first period of time, and then can be administered via another route (e.g., subcutaneously) for a second period of time. For example, a composition containing an MANP can be intravenously administered to a mammal (e.g., a human) at a dose of about 1 pmol/kg/minute to about 500 nmol/kg/minute for one hour to seven days (e.g., one to two hours, two to four hours, four to six hours, six to eight hours, eight to 12 hours, 12 to 24 hours, 24 to 48 hours, 48 to 36 hours, three to four days, four to five days, five to six days, or six to seven days), and subsequently can be subcutaneously administered to the mammal at a dose of about 0.01 ng/kg to about 100 mg/kg, one to three times daily, for five to 30 days or more (e.g., five to seven, seven to 10, 10 to 14, 14 to 21, 21 to 28, 28 to 30, or more than 30 days).

An effective amount of an MANP (or a nucleic acid encoding an MANP) and a diuretic agent administered to a mammal is an amount that is sufficient to alter a selected parameter (e.g., blood pressure) by at least 10%. For example, in some embodiments, an "effective amount" of an MANP and a diuretic agent is an amount that is sufficient to increase natriuresis and/or diuresis (or to increase or decrease a characteristic of natriuresis and/or diuresis such as plasma cGMP levels, urinary cGMP excretion, net renal cGMP generation, urine flow, urinary sodium excretion, urinary potassium excretion, hematocrit, plasma BNP immunoreactivity, renal blood flow, plasma ANP immunoreactivity, renal vascular resistance, proximal and distal fractional reabsorption of sodium, mean arterial pressure, pulmonary wedge capillary pressure, right atrial pressure, pulmonary arterial pressure, plasma renin activity, plasma angiotensin II levels, plasma aldosterone levels, renal perfusion pressure, and systemic vascular resistance) by at least 10% (e.g., at least 10%, at least 15%, at least 20%, at least 25%, at least 30%, at least 40%, at least 50%, at least 60%, at least 70%, at least 75%, at least 80%, at least 85%, at least 90%, at least 95%, at least 99%, or at least 100%), as compared to the level of the same parameter prior to treatment, or as compared to the level of the parameter in a control, untreated mammal. Thus, in some cases, an effective amount of an MANP and a diuretic agent is an amount that reduces blood pressure in a mammal identified as having hypertension (e.g., RH) by at least 10% (e.g., at least 15%, at least 20%, at least 25%, at least 30%, at least 40%, or at least 50%) as compared to the blood pressure in the mammal prior to administration of the MANP and diuretic agent, or without administration of the MANP and diuretic agent, or as compared to "normal" blood pressure in a control, untreated mammal. An effective amount of an MANP also can be an amount sufficient to potentiate the effectiveness of a diuretic agent, such that a beneficial effect of the diuretic (e.g., reducing blood pressure) is increased (e.g., by at about least 5%, at least about 10%, at least about 15%, at least about 20%, at least about 30%, at least about 40%, or at least about 50%) beyond the effect observed when the same amount of the diuretic agent is administered without the MANP. An effective amount of an MANP also can reduce a detrimental effect of a diuretic agent (e.g., renin-angiotensin-aldosterone activation) by at least about 5% (e.g., at least about 10%, at least about 15%, at least about 20%, at least about 30%, at least about 40%, or at least about 50%) as compared to the detrimental effect observed when the diuretic is administered without the MANP.

In some embodiments, the amount and frequency of MANP and diuretic agent administration to a mammal can be titrated in order to, for example, identify a dosage that is most effective to treat hypertension while having the least amount of adverse effects. If a mammal fails to respond to a particular amount, then the amount can be increased by, for example, two-fold, three-fold, five-fold, or ten-fold. After receiving this higher concentration, the mammal can be monitored for both responsiveness to the treatment and toxicity symptoms, and adjustments in the dosage and/or frequency of administration can be made accordingly. The effective amount can remain constant or can be adjusted as a sliding scale or variable dose depending on the mammal's response to treatment.

The frequency of administration can be any frequency that reduces a symptom of, for example, cardiovascular or cardiorenal disease within a mammal, without producing significant toxicity in the mammal. For example, the frequency of administration can be from about four times a day to about once every other month, or from about once a day to about once a month, or from about once every other day to about once a week. In addition, the frequency of administration can remain constant or can be variable during the duration of treatment. As with the effective amount, various factors can influence the actual frequency of administration used for a particular application. For example, the effective amount, duration of treatment, route of administration, and severity of renal condition may require an increase or decrease in administration frequency.

An effective duration of administration can be any duration that reduces hypertension or a symptom of cardiorenal disease within a mammal without producing significant toxicity in the mammal. The effective duration can vary from one to several days, to several weeks, months, or years. In general, the effective duration can range in duration from several days to several months. For example, an effective duration can range from about one to two weeks to about 36 months, or even longer. In some cases, treatment may last throughout an individual mammal's lifetime. Multiple factors can influence the actual effective duration used for a particular treatment regimen. For example, an effective duration can vary with the frequency of administration, amount administered, route of administration, and severity of a condition.

After administering an MANP and a diuretic agent as described herein to a mammal, the mammal can be monitored to determine whether or not the disorder has improved. For example, a mammal can be assessed after treatment to determine whether or not one or more symptoms of the mammal's disorder have been reduced (e.g., whether the mammal's blood pressure has decreased). If a mammal fails to respond to particular doses of an MANP and a diuretic agent, then the amounts of one or both can be increased by, for example, two-fold, three-fold, five-fold, or ten-fold. After receiving this higher concentration, the mammal can be monitored for both responsiveness to the treatment and toxicity symptoms, and adjustments made accordingly. The effective amount can remain constant or can be adjusted as a sliding scale or variable dose depending on the mammal's response to treatment.

In some cases, a method as provided herein can include administering, to a mammal, one or more compositions in which the sole active ingredients are a diuretic agent and an MANP. In such cases, for example, the administering can consist of administering a composition in which the sole active ingredients are an MANP and a diuretic, or the administering can consist of administering a first composition in which the sole active ingredient is an MANP and a second composition in which the sole active ingredient is a diuretic agent.

In some cases, a method as provided herein can include administering, to a mammal, one or more compositions in which the sole active ingredients are a diuretic agent, an MANP, and either an ACE inhibitor, an ARB, or a CCB. In such cases, for example, the administering can consist of administering a composition in which the sole active ingredients are an MANP, a diuretic, and an ACE inhibitor; or an MANP, a diuretic, and an ARB; or an MANP, a diuretic, and a CCB, or the administering can consist of administering a first composition in which the sole active ingredient is an MANP, a second composition in which the sole active ingredient is a diuretic agent, and a third composition in which the sole active ingredient is an ACE inhibitor, an ARB, or a CCB. It is noted that in some cases, two of the three active ingredients (e.g., the MANP inhibitor and the diuretic, the MANP and the ACE inhibitor, ARB, or CCB, or the diuretic and the ACE inhibitor, ARB, or CCB) can be administered in one composition and the third active ingredient can be administered in a separate composition.

In some cases, a method as provided herein can include administering, to a mammal, one or more compositions in which the sole active ingredients are a diuretic agent, an MANP, an ACE inhibitor or an ARB, and a CCB. In such cases, for example, the administering can consist of administering a composition in which the sole active ingredients are an MANP, a diuretic, an ACE inhibitor or an ARB, and a CCB, or the administering can consist of administering a first composition in which the sole active ingredient is an MANP, a second composition in which the sole active ingredient is a diuretic agent, a third composition in which the sole active ingredient is an ACE inhibitor or an ARB, and a fourth composition in which the sole active ingredient is a CCB. It is noted that in some cases, two of the active ingredients (e.g., the MANP and the diuretic, the MANP and the ACE inhibitor or ARB, the MANP and the CCB, the diuretic and the ACE inhibitor or ARB, the diuretic and the CCB, or the ACE inhibitor or ARB and the CCB) can be administered in one composition and the other two active ingredients can be administered in a separate composition. Further, in some cases, three of the active ingredients (e.g., the MANP, the diuretic, and the ACE inhibitor or ARB; the MANP, the diuretic, and the CCB; the MANP, the ACE inhibitor or ARB, and the CCB; or the diuretic, the ACE inhibitor or ARB, and the CCB) can be administered in one composition and the remaining active ingredient can be administered in a separate composition.

In some cases, the methods provided herein can include identifying a mammal as having hypertension (e.g., RH), and then administering an MANP and a diuretic agent (e.g., Fs) to the mammal. Having the ability to identify mammals as having hypertension or RH can allow those mammals to be properly identified and treated in an effective and reliable manner with a combination of an MANP and a diuretic agent. For example, the RH treatments described herein (e.g., a combination of an MANP and Fs) can be used to treat hypertension patients identified as having RH.

The invention will be further described in the following examples, which do not limit the scope of the invention described in the claims.

### EXAMPLES

### Example 1 - Effects of MANP and Fs in rats

Studies were conducted to examine the individual and combined effects of MANP (SEQ ID NO:3) and Fs on BP and aldosterone stimulation in spontaneously hypertensive rats (SHR), the most studied and widely used genetic model of human essential hypertension (Lerman et al. (2019) Hypertension 73(6):e87-e120). SHR (n=32) were randomized into ten groups:
Group 1 - treated with vehicle saline (Veh);
Group 2 - treated with 1 mg/kg Fs alone (Fs1);
Group 3 - treated with 5 mg/kg Fs alone (Fs5);
Group 4 - treated with 10 mg/kg Fs alone (Fs10);
Group 5 - treated with 100 pmol/kg/min MANP alone (MANP100);
Group 6 - treated with 300 pmol/kg/min MANP alone (MANP300);
Group 7 - treated with 600 pmol/kg/min MANP alone (MANP600),
Group 8 - treated with Fs5+MANP300;
Group 9 - treated with Fs10+MANP300; and
Group 10 - treated with Fs10+MANP600.
Fs was given as a single bolus that, in groups 8, 9, and 10, was followed by a 60-minute infusion of MANP.

Mean arterial pressure (MAP, mmHg) was reduced by treatment with Fs1 (Δ = -40±9*), Fs5 (Δ = -55±8*), Fs10 (Δ = -63+11%*), MANP 300 (Δ = -70±19*), and MANP600 (Δ = -93+20*^{&#}) alone; a greater effect was observed in the combined Fs5+MANP300 (Δ = -90±15*^{&#}), Fs10+MANP300 (Δ = -93±6*^{&#}), and Fs10+MANP600 (Δ = -95±9*^{&#}) treatment groups (**FIGS. 2A-2C****).** Reductions in BP in the Fs, MANP, and Fs + MANP groups were associated with enhanced sodium excretion and urinary flow (*p<0.05), but no significant difference in natriuresis or diuresis was observed between the Fs and Fs+MANP groups. Plasma cGMP was significantly increased in the MANP and MANP+Fs groups, but was not changed with Fs alone **(****FIGS. 3A-3C****).** Plasma aldosterone tended to increase with low and high dose Fs, and the increase was significantly attenuated in the Fs+MANP groups (p<0.05) **(****FIG. 4****).** For the MANP+Fs groups in particular, greater decreases in BP were correlated with greater increases in cGMP **(****FIG. 5****).**

Thus, MANP+Fs had more potent BP lowering effects than Fs alone, and also suppressed Fs-induced activation of aldosterone. Fs and MANP both induce diuresis and natriuresis, while MANP also may mediate vasorelaxation, providing a possible explanation for the more potent anti-hypertensive action of Fs+MANP. These studies therefore suggested that MANP is an effective add-on drug for the treatment of RH, in order to potentiate the anti-hypertensive actions of Fs.

### Example 2 - Effects of MANP and Fs in HEK293 cells and rats

The studies of MANP were extended to further investigate its acute dose dependent cardiorenal and neurohumoral actions in SHR, to confirm its direct cGMP activating actions in HEK293 cells overexpressing pGC-A, and to examine MANP-mediated cGMP activation in human primary vascular cells, which are important organ damaging targets of hypertension. These included human aortic smooth muscle cells and human aortic endothelial cells, both of which naturally express pGC-A.

### Methods

*Cell Culture:* HEK293 cells were transfected with pGC-A (cDNA clones from Origene, Rockville, MD) using Lipofectamine (Invitrogen, Grand Island, NY). Transfected cells were maintained in Dulbecco's modified Eagle's medium supplemented with 10% fetal bovine serum (FBS) (Gibco), 100 U/mL penicillin, 100 U/mL streptomycin, and 250 µg/mL G418 (Geneticin) antibiotic. HAECs (Lonza, Walkersville, MD) were cultured in EBM-2 medium (Lonza) containing EGM-2 growth factors and supplements (Lonza) and 10% FBS (Gibco). HASMCs (ScienCell, San Diego, CA) were cultured in smooth muscle cell medium (SMCM) (ScienCell) containing SMC growth supplement (SMCGS), penicillin/streptomycin (ScienCell) and FBS (ScienCell).

*Intracellular cGMP:* Cells were treated at 80% to 90% confluency. Briefly, cells were incubated in a buffer containing 20 mmol/L N-[2-hydroxyethyl]piperazine-N'-[2-ethanesulfonic acid], 0.1% bovine serum albumin, and 0.5 mmol/L 3-isobutyl-1-methylzanthine (Sigma, St. Louis, MO). Treated cells received no treatment (control) or MANP (SEQ ID NO:3; 10⁻⁸ M, 10⁻⁷ M, or 10⁻⁶ M) for 10 minutes. Experiments were performed in triplicate for each concentration of MANP or control and then cell lysates were collected. The samples were assayed using direct cGMP ELISA kit (Enzo Life Sciences, Farmingdale, NY) according to the manufacturer's protocol. Total protein concentration in the samples was quantified using a BCA protein assay kit (Thermo Scientific, Rockford, IL). Results are expressed as picomoles of cGMP per milligram of total protein.

*Acute Study Protocol in SHR:* The cardiorenal and neurohumoral actions of MANP, Fs, or their combination were investigated in the genetic model of hypertension in male SHR (n=6 in each group). Rats were anesthetized in an induction chamber with inhaled Isoflurane 2.5%. During this brief state of anesthesia, the rats were administered IP Inactin 100 mg/kg to maintain anesthesia. An oxygen mask was used until the end of the experiment. The carotid artery was cannulated for arterial pressure measurements and the right jugular vein was cannulated for infusions. Pressures were recorded and analyzed digitally (Sonometrics, London, Ontario, Canada). Via a small incision in the lower abdominal wall, the bladder was cannulated for urine sampling.

Immediately after preparation, a 30-minute equilibration period was started with intravenous inulin infusion. A 15-minute baseline clearance was performed that consisted of hemodynamic measurements and arterial blood sampling. After baseline measurements, rats were randomly assigned to ten groups:
Group 1 - treated with MANP100;
Group 2 - treated with MANP300;
Group 3 - treated with MANP600;
Group 4 - treated with Fs1;
Group 5 - treated with Fs5;
Group 6 - treated with Fs10;
Group 7 - treated with MANP300 + Fs5;
Group 8 - treated with MANP300 + Fs10;
Group 9 - treated with MANP600 + Fs10; and
Group 10 - treated with inulin, normal saline, or inulin and normal saline.
For Groups 1-3, after a 15-minute lead-in of MANP infusion, MANP was continuously infused for a total of 60 minutes. Animals in Groups 4-6 received a single intravenous (IV) bolus administration of Fs. MANP+Fs Groups 7-9 received a single IV bolus injection of Fs followed by a 15-minute lead-in infusion of MANP with a further 60-minute continuous infusion of MANP at the same dose. The control animals (Group 10) received a continuous inulin infusion as an MANP control, a single IV bolus injection of 0.9% normal saline as a Fs control, or the combination of a single IV bolus of 0.9% normal saline and continuous inulin infusion as a control for MANP+Fs treatment.

*Neurohormonal and Electrolyte Analysis:* Plasma ANP was measured by radioimmunoassay (Phoenix Pharmaceuticals) as an assessment of MANP, as the ANP RIA fully detects MANP (Burnett et al. (1986) Science 231(4742):1145-1147). Plasma and urinary cGMP (Enzo Life Sciences) and aldosterone (DRG International, Springfield, NJ) were determined by ELISA. Inulin concentrations were measured using the anthrone method, as described elsewhere (Fuhr et al. (1955) Klin. Wochenschr 33:729-730). GFR was calculated by inulin clearance. Electrolytes were measured by flame photometry (IL943, Instrumentation Laboratory, UK). All measurements were performed according to manufacturer instructions.

*Statistical Analysis:* Values are expressed as mean ± standard error of mean (SEM). The comparison of independent groups was performed by Student's t-test. One-way ANOVA test followed by Dunnett's post-hoc test was used for comparisons within groups. Two-way ANOVA test was used for comparisons between groups, followed by a Bonferroni post-hoc test (Statistica 8.0). Statistical significance was defined as p-value <0.05.

### Results

*In Vitro cGMP Activation in HEK293 Overexpressed Human pGC-A and in Primary Human Aortic Endothelial Cells and Human Aortic Smooth Muscle Cells:* **FIG. 6A** presents results from *in vitro* measurement of cGMP production after MANP treatment of HEK293 cells transfected with human pGC-A receptor. The level of cGMP increased with gradually increasing concentrations of MANP. MANP significantly increased cGMP levels, compared with the no treatment control group. Increasing doses of MANP also resulted in significant increases in cGMP levels in HAECs and HASMCs **(****FIGS. 6B and 6C****).** Similar to HEK 293 cells, there was no significant effect of no treatment.

*In Vivo Study - Systemic Hemodynamics:* Arterial BP in SHR for the MANP, Fs, MANP+Fs, and control groups at the baseline are presented in **TABLE 2.** Effects on BP are illustrated in **FIGS. 7A-7C** **and** **8****.** MANP300 and MANP600 significantly decreased BP when compared to the control groups **(****FIG. 7A****).** At 60 minutes, the greatest decrease in BP was in the high dose (MANP600) group **(****FIG. 8****).** All three doses of Fs significantly reduced BP compared with controls **(****FIGS. 7B** **and** **8****).** However, MANP600 alone had a significantly greater antihypertensive effect than Fs1 or Fs5 alone **(****FIG. 8****).** In the combination therapy groups, the addition of MANP300 to Fs5 or Fs10, as well as the addition of MANP600 to Fs10, significantly reduced BP when compared with Fs alone (single-drug therapy) (p<0.05 for MANP300+Fs5 vs. Fs5 alone, MANP300+ Fs10 vs. Fs10 alone, and MANP600+Fs10 vs. Fs10 alone) **(****FIGS. 7C** **and** **8****).** Thus, combination therapy MANP+Fs significantly potentiated the antihypertensive effects of Fs.

*In Vivo Study* - *Neurohumoral Analysis:* Data for plasma cGMP levels in the MANP, Fs, MANP+Fs, and control groups at baseline are summarized in **TABLE 2.** Plasma cGMP was determined at 3 time points (baseline, 30 minutes, and 60 minutes), and increased in a dose-dependent manner after treatment with MANP300 and MANP600 as compared to the control groups **(****FIG. 9A****).** In comparison, Fs did not change plasma cGMP **(****FIG. 9B****),** but plasma cGMP was markedly increased in the MANP+Fs group **(****FIG. 9C****).**

**TABLE 2**

| *Baseline values of BP and plasma cGMP in MANP, Fs, MANP+Fs, and vehicle groups* | | |
|---|---|---|
| **Treatment** | **Baseline BP (mmHg)** | **Baseline cGMP (pM/mL)** |
| Control (vehicle) | 163±10 | 36±5 |
| MANP 100 | 166±11 | 42±6 |
| MANP 300 | 165±9 | 48±3 |
| MANP 600 | 169±7 | 57±3 |
| Fs 1 | 165±5 | 56±2 |
| Fs 5 | 168±11 | 51±3 |
| Fs 10 | 169±8 | 48±5 |
| MANP 300+Fs5 | 169±8 | 43±9 |
| MANP 300+Fs10 | 168±9 | 43±6 |
| MANP 600+Fs10 | 170±7 | 41±1 |

Urinary cGMP excretion was significantly increased after MANP300 and MANP600 administration, in a dose-dependent manner, and also was significantly increased after administration of MANP300+Fs5, MANP300+Fs10 and MANP600+Fs10 when compared to controls **(****FIG. 10****).** There was no significant increase in the Fs groups, which did not differ from vehicle (control) groups **(****FIG. 10****).**

Assessment of aldosterone is illustrated in **FIG. 11****.** MANP reduced aldosterone at doses of 100 pmol/kg/minute and 300 pmol/kg/minute, but not at 600 pmol/kg/minute. There were significantly higher levels of aldosterone in the Fs5 and Fs10 groups compared to the control, MANP100, and MANP300 groups. In the combination groups, aldosterone was significantly decreased in the MANP300+Fs5 group compared to Fs5 alone, as well as in the MANP300+Fs10 and MANP600+Fs10 groups as compared to Fs10 alone. Therefore, treatment with MANP+Fs was able to suppress Fs-induced activation of aldosterone.

MANP infusion alone or in combination with Fs resulted in significant increases in plasma ANP immunoreactivity as a measure of MANP immunoreactivity **(TABLE 3).** Among neurohumoral factors, plasma ANP levels significantly correlated with plasma cGMP (r=0.75, p<0.001) **(****FIG. 12****).** Among the hemodynamic parameters, plasma cGMP concentrations significantly correlated with BP (r=-0.65, p<0.001) **(****FIG. 13****).**

*In Vivo Study - Renal Function:* Effects on GFR, urinary sodium excretion (UNaV) and water excretion (UV) are plotted in **FIGS. 14A-14C****.** GFR was significantly lower in the Fs groups compared to the no treatment control groups, but GFR was increased in the MANP300+Fs10 and MANP600+Fs10 groups as compared to the Fs groups **(****FIG. 14A****).** There was a significant increase in both urinary sodium excretion **(****FIG. 14B****)** and water excretion **(****FIG. 14C****)** after administration of MANP alone, as well as in combination with Fs.

**TABLE 3**

| *Plasma ANP levels after 60-min treatment with MANP, Fs, MANP*+*Fs*, *or vehicle* | |
|---|---|
| **Treatment** | **ANP (pG/mL)** |
| Vehicle | 19±1 |
| MANP 100 | 430±99 |
| MANP 300 | 2577±568 |
| MANP 600 | 20611±3041 |
| Fs 1 | 14±1 |
| Fs 5 | 16±3 |
| Fs 10 | 19±3 |
| MANP 300+Fs5 | 7089±507 |
| MANP 300+Fs10 | 6362±417 |
| MANP 600+Fs10 | 11561±1259 |

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. An M-atrial natriuretic peptide (MANP) and a diuretic agent for use in a method for potentiating one or more beneficial effects of said diuretic agent, comprising:
administering to said mammal said MANP, and
administering to said mammal said diuretic agent,
wherein said diuretic agent is selected from the group consisting of: chlorthalidone, chlorothiazide, hydrochlorothiazide, indapamide, metolazone, amiloride, bumetanide, spironolactone, triamterene, and furosemide.

2. The MANP and the diuretic agent for use of claim 1, wherein said mammal is identified as having hypertension, optionally wherein said hypertension is resistant hypertension (RH).

3. The MANP and the diuretic agent for use of any one of claims 1 to 2, wherein said MANP has the amino acid sequence set forth in SEQ ID NO:3 or the amino acid sequence set forth in any one of SEQ ID NOS:5 to 14.

4. The MANP and the diuretic agent for use of any one of claims 1 to 3, wherein said diuretic agent is furosemide.

5. The MANP and the diuretic agent for use of any one of claims 1 to 4, wherein said mammal is a human.

6. The MANP and the diuretic agent for use of any one of claims 1 to 5, wherein said MANP is administered subcutaneously at a dose of about 0.1 ng/kg to about 10 mg/kg.

7. The MANP and the diuretic agent for use of any one of claims 1 to 5, wherein said MANP is administered intravenously at a dose of about 10 pmol/kg/minute to about 100 nmol/kg/minute.

8. The MANP and the diuretic agent for use of any one of claims 1 to 5, wherein said MANP is administered intravenously at a dose of about 10 pmol/kg/minute to about 100 nmol/kg/minute, and subsequently is administered subcutaneously at a dose of about 0.1 ng/kg to about 100 mg/kg.

9. The MANP and the diuretic agent for use of any one of claims 1 to 5, wherein said MANP and said diuretic agent are administered intravenously, and wherein said MANP is administered intravenously at a dose of about 10 pmol/kg/minute to about 100 nmol/kg/minute.

10. The MANP and the diuretic agent for use of any one of claims 1 to 5, wherein said MANP and said diuretic agent are administered subcutaneously, and wherein said MANP is administered subcutaneously at a dose of about 1 µg/kg to about 10 µg/kg.

11. The MANP and the diuretic agent for use of any one of claims 1 to 5, wherein said MANP and said diuretic agent are administered intravenously, and subsequently are administered subcutaneously, and wherein said MANP is administered intravenously at a dose of about 10 pmol/kg/minute to about 100 nmol/kg/minute, and subsequently is administered subcutaneously at a dose of about 1 µg/kg to about 10 µg/kg.

12. The MANP and the diuretic agent for use of any one of claims 1 to 8, wherein said diuretic agent is administered orally, intravenously, or subcutaneously.

13. The MANP and the diuretic agent for use of any one of claims 1 to 8, wherein said MANP and said diuretic agent are administered simultaneously in the same composition.

14. The MANP and the diuretic agent for use of any one of claims 1 to 13, wherein said one or more beneficial effects comprise a reduction in blood pressure in the mammal.
